(19) Europäisches Patentamt
European Patent Office
Office européen des brevets

(11) **EP 2 462 978 A1**

(12) **EUROPEAN PATENT APPLICATION**
published in accordance with Art. 153(4) EPC

(43) Date of publication:
**13.06.2012 Bulletin 2012/24**

(51) Int Cl.:
***A61M 37/00*** *(2006.01)*

(21) Application number: **10806230.8**

(22) Date of filing: **04.08.2010**

(86) International application number:
**PCT/JP2010/004903**

(87) International publication number:
**WO 2011/016230 (10.02.2011 Gazette 2011/06)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **07.08.2009 JP 2009184683
02.09.2009 JP 2009202328**

(71) Applicant: **Medrx Co., Ltd.
Kagawa 769-2712 (JP)**

(72) Inventors:
• **KOBAYASHI, Katsunori
Higashikagawa-shi
Kagawa 769-2712 (JP)**
• **HAMAMOTO, Hidetoshi
Higashikagawa-shi
Kagawa 769-2712 (JP)**

(74) Representative: **Jackson, Martin Peter
J A Kemp
14 South Square
Gray's Inn
London WC1R 5JJ (GB)**

(54) **APPLICATOR DEVICE OF PINHOLDER TYPE MICRONEEDLE**

(57) Provided is a microneedle device which protects a microneedle, has an easily portable shape, is free from such problems as breakage of small needles in the step of puncturing the skin with the microneedle, and ensures appropriate skin puncture to administer a drug. By studying the relationship between the device to be pressed to the skin and the height of the elevation of the skin surface during the pressing, it is found that there is a positive correlation between the placement and interval of skin fixing members and the height of the elevation of the skin. It is furthermore found that the elevated skin surface is always parallel to the flat plate of the device. This reveals that since the microneedle installed in the device can be inserted at right angle to the skin surface, the skin can reliably be punctuated avoiding the breakage etc. of the small needles of the microneedle. Thus, a microneedle patch which is easily portable and ensures convenient drug administration to the skin can be produced.

Fig. 20

**Description**

**TECHNICAL FIELD**

The present invention relates to an applicator used to puncture skin with a pin-frog-shaped (*kenzan*-shaped) microneedle effectively. Also, the present invention relates to a method for plastering a patch formulation using the pin-frog-shaped microneedle.

**BACKGROUND ART**

Transdermal administration of drugs has been normally achieved by using formulations for transdermal administration in the form of solutions or ointments to be applied or plastered to the skin surface. The skin, in the case of human, normally comprises a number of tissues: the stratum corneum (horny layer) having a laminar structure in a thickness of 10 to 30 $\mu$m, the epidermal tissue layer in a thickness of about 70 $\mu$m, and the dermal tissue layer in a thickness of about 2 mm.

The stratum corneum, which is a laminar structure constituting the outermost layer of the skin, functions as a barrier to prevent various drugs from penetrating the skin. Generally, about 50 to 90% of the barrier action of the skin is attributed to the stratum corneum. The epidermal layer does not contribute to the barrier action as great as the stratum corneum; however it accounts for the remaining about 10% or more of the skin barrier action. Meanwhile, the dermal layer has an extensive capillary network in the vicinity of the junction between the dermal layer and the epidermal layer. Once a drug reaches the depth of the dermis, the drug quickly diffuses into deeper tissues (hair follicles, muscles and the like) through the capillary network. Then, the drug is systematically diffused through the blood circulation from the capillaries.

Currently, there have been developed various formulations for transdermal administration in the form of solutions or ointments to be applied or plastered to the skin surface. However, due to the barrier action of the stratum corneum described above, absorption of drug-effective components is insufficient. For example, it is said that, even formulations for transdermal administration of indomethacin, which are considered to exhibit high transdermal absorption rate, enable only about 5% of the total amount of indomethacin to be absorbed transdermally.

Accordingly, as one method to increase the skin permeability of a drug, as shown in Patent Document 1, it has been tried to topically destroy the stratum corneum using small needles (microneedle or microsyringe) to forcibly deliver the drug into the dermal layer.

The small needles used for this purpose have preferably 30 $\mu$m or more in length in order that they reach the dermal layer, and the small needles are considered to require a base for supporting the needles. Since the small needles do not reach the dermal layer where nerve terminals are located, they do not cause pain. Therefore, there is an advantage of administering a drug without causing fear to children, etc.

While a wide variety of methods for producing the microneedle have been studied, there also have been studied devices (i.e., assistive tool) for puncturing skin with the microneedle effectively. These devices employ a method of using impact force of a spring or the like to puncture skin with the microneedle (Patent Documents 2, 3 and 5).

These puncturing methods of the microneedle using impact force, however, may cause easy breakage of the small needles of the microneedle in the step of puncturing the skin and also cause excessive damage to the skin surface. Therefore, there is a demand for devices that cause less impact and can puncture the skin softly. Accordingly, an array device in the form of a syringe has been reported, in which a piston is inserted into a tube and pressed by hand to puncture the skin slowly (Patent Document 4).

Patent Document 1: JP-A-2006-149818
Patent Document 2: JP-A-2008-520369
Patent Document 3: JP-A-2008-543527
Patent Document 4: WO 2008/069566
Patent Document 5: JP-A-2008-535587

**DISCLOSURE OF THE INVENTION**

**PROBLEMS TO BE SOLVED BY THE INVENTION**

The present invention provides an applicator (insertion tool) for a microneedle array, which is convenient and portable. In particular, the microneedle made of a biodegradable resin (small needle parts) is not sufficiently strong, and therefore in many cases, when they approach the skin at angles other than vertical, they may be broken at the time of contact with the skin surface and cannot be inserted into the skin or may be broken at the time of insertion and remain embedded in the skin. Thus, there is provided an applicator which enables the microneedle made of a biodegradable resin to be surely and easily inserted into skin.

Further, the present invention provides an applicator on which a tape-like chip (an adhesive tape in which a microneedle is stored) is placed, wherein when the skin is punctured with the microneedle by using this applicator and then the applicator is removed therefrom, the pin-frog-shaped microneedle is adhered to the skin with the tape and remains on the skin.

## MEANS FOR SOLVING THE PROBLEMS

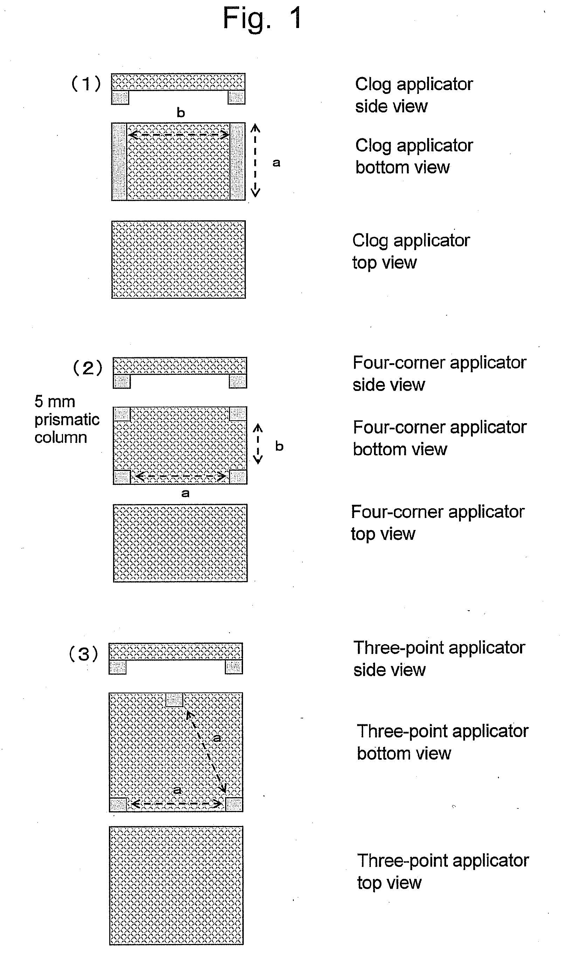

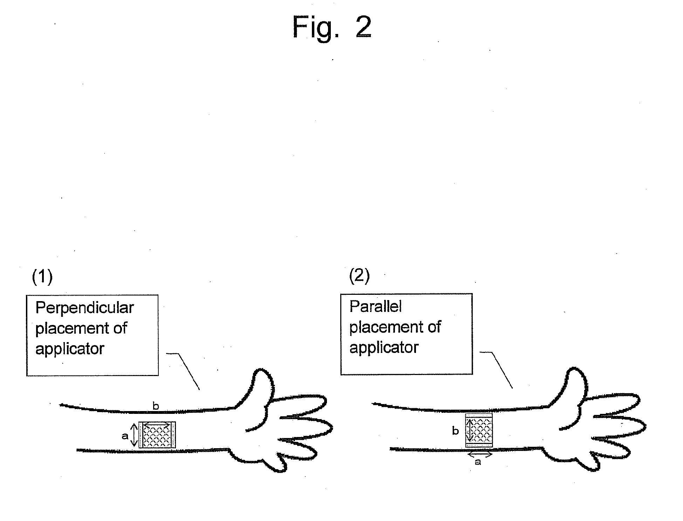

When the skin surface was pressed with a tubular hard member, it was seen that the skin inside the tubular member was slightly elevated and slightly entered into the tubular member. Observing the way the skin was elevated and protruded into the tubular member in detail, the following points became apparent. Here, a tubular member having a cut end perpendicular to the axis direction of the tubular member was used, and the cross-section thereof was pressed against the skin surface. At that time, the present inventors found out that, even when the pressing angle of the tubular member against the skin (an angle between the axis direction of the tubular member and the flat skin surface) was not a right angle, or even when the tubular member was shifted or moved slightly, this hardly affected the elevation of the skin surface inside the tubular member. Also, they found out that the direction of the elevation of the skin surface was approximately the same as the axis direction (PCT/JP2009/53749). Accordingly, the present inventors made three types of applicators having skin fixing members shown in Fig. 1, in place of the tubular member. Namely, clog-shaped (Japanese "geta"-shaped) applicator (a device having, as skin fixing members, prismatic columns, semicircular columns, etc. at both ends of a flat plate) as shown in Fig. 1(1); a four-corner applicator (a device having, as skin fixing members, prismatic columns, circular columns, hemispheres, etc. at four corners of a flat plate) as shown in Fig. 1(2); and a three-point applicator (a device having, as skin fixing members, three prismatic columns, circular columns, hemispheres, etc. at a peripheral portion of a flat plate) as shown in Fig. 1(3). On the underside (the face of a flat plate to which prismatic columns etc. are attached) of each of these applicators, were placed microneedles made of a biodegradable resin. In the case where such an applicator (e.g. the clog-shaped applicator in (1)) is placed in two directions with respect to the arm and pressed against the arm as shown in Fig. 2, the skin is elevated in the space defined between the skin fixing members (space defined by the distance a and the distance b) during the pressing of the applicator.

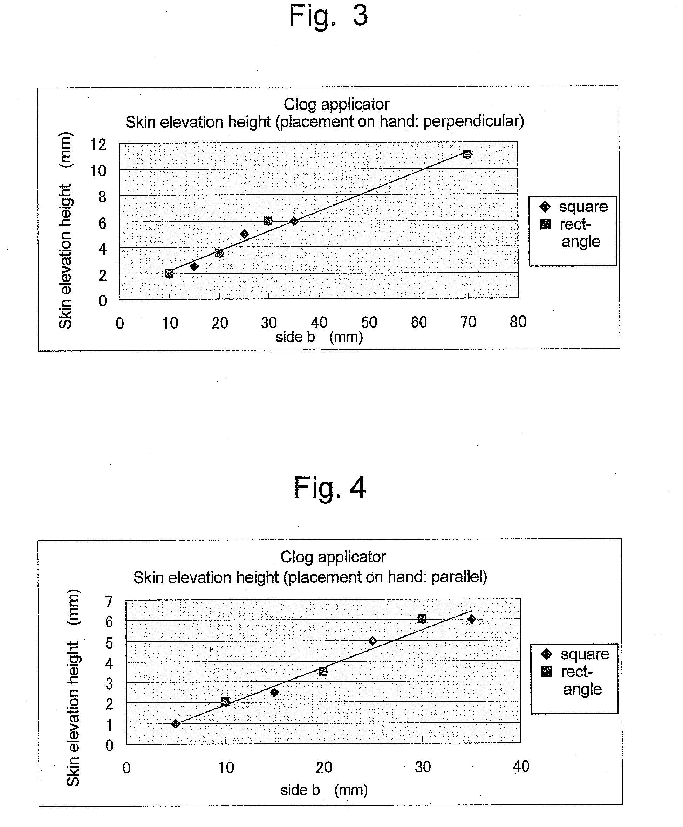

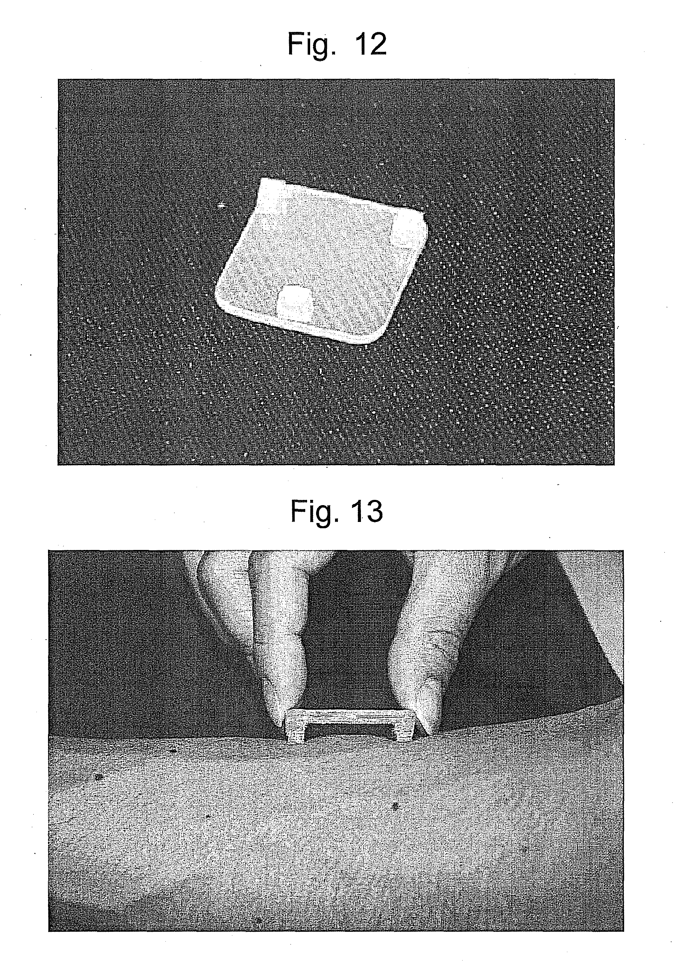

The present inventors studied a correlation between the distance b and the height of elevation of the skin during the pressing of the applicator. First, the applicator was placed on the skin and pressed down about 12 mm from the original position, as shown in Fig. 13. Then, the present inventors measured the height of the elevation of the skin (the distance between the skin fixed by the skin fixing members and the top of the elevated skin) in the space defined between the skin fixing members (space defined by the distance a and the distance b). As a result, there was shown a correlation between the distance b and the height of elevation of the skin, as shown in Figs. 3 and 4.

Next, the present inventors prepared a tool in order to assess the manner the skin is elevated (Fig. 16), and they studied how the skin surface changes and elevates depending on the size of the device and the position of the device where force is applied, as shown in Fig. 17. As a result, they found out that, as shown in Fig. 18 and Tables 6 and 7, the elevated skin surface rose perpendicularly to the device regardless of the size of the device and the position of the device where force was applied, thereby the pin-frog-shaped microneedle that had been placed on the device was inserted into the skin surface perpendicularly.

In addition, they found out that it is preferred to locate the skin fixing members point-symmetrically with the microneedle placed at the center or to locate the fixing members at corners of a regular polygon with the microneedle being at the center. That is, when the skin fixing members are located in the positions described above with the microneedle being at the center, the skin surface is elevated perpendicularly during the pressing of the applicator.

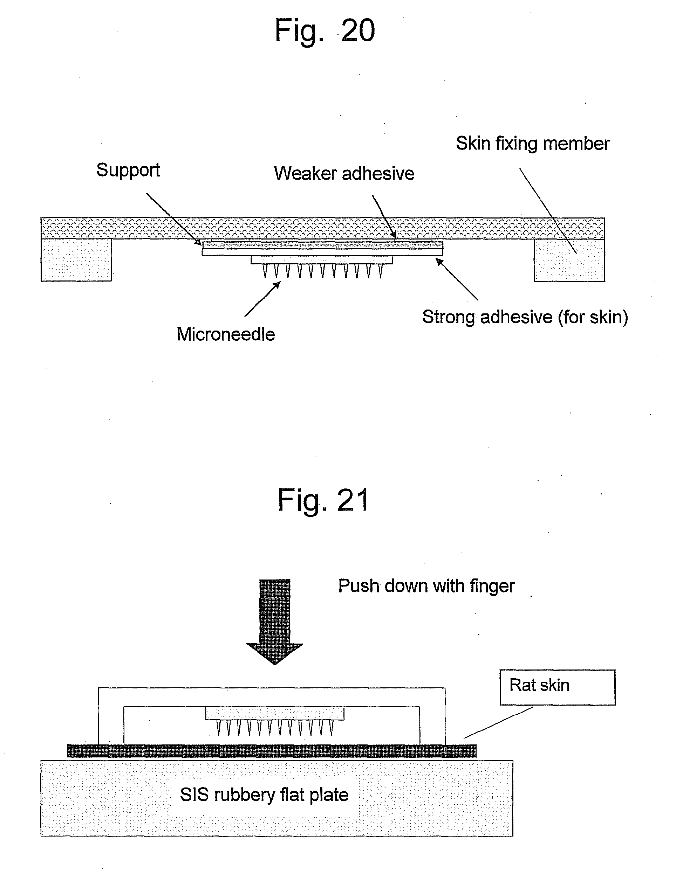

Further, the present inventors prepared a support to both faces of which an adhesive was applied, as shown in Fig. 20, and placed a microneedle on the stronger adhesive to prepare a tape-like chip (a cataplasm that holds a microneedle). Then, they proceeded and studied the following: the tape-like chip was stored and fixed in the applicator etc. with the weaker adhesive and pressed against the skin to adhere the microneedle to the skin together with the tape. As a result, the present inventors found out that, when the difference of the adhesive strength (adhesion) between the adhesives used in both faces of the support is 10 to 15% or more, the tape-like chip is detached from the applicator etc. and remained on the skin while being fixed and attached to the skin. Further, they found out that, when the applicator etc. is pulled diagonally and detached therefrom, the tape-like chip remains on the skin more easily. Also, they found out that, depending on the shape of the weaker adhesive, the tape-like chip remains on the skin more easily. Based on these findings, the present inventors have completed the present invention.

The present invention has been completed based on these findings and upon further studies, and a summary thereof is as follows.

(1) A microneedle device for puncturing skin with a microneedle, **characterized in that**

a) the device comprises two or more skin fixing members, for fixing the skin, at a peripheral portion of a flat plate,
b) the distance between the fixing members is 5 to 70 mm,
c) the fixing members each have a prismatic column shape, circular column shape, barrel-roof shape (semicircular column shape), or hemispherical shape, and
d) the microneedle is placed on the flat plate at a central portion between/among the fixing members.

(2) The microneedle device according to (1), wherein the microneedle is placed on the surface of the flat plate, on which the fixing members are present.
(3) The microneedle device according to (1) or (2), wherein the fixing members are two rectangular parallelepiped members (as a clog-shaped applicator).
(4) The microneedle device according to any of (1) to (3), wherein the fixing members are four hemispherical-shaped members (as a four-corner applicator).
(5) The microneedle device according to any of (1) to (3), wherein the fixing members are three hemispherical-shaped members (as a three-point applicator).

(6) The microneedle device according to (3), wherein tip portions of small needles of the microneedle are positioned within the device and less than tip surfaces of the skin fixing members, and the difference of the height (distance) between the tips of the small needles of the microneedle and the tip surfaces of the fixing members is equal to "the distance between two rectangular parallelepipeds" $\times$ 0 to 16%.
(7) The microneedle device according to (4), wherein tip portions of small needles of the microneedle are positioned within the device and less than tip surfaces of the skin fixing members, and the difference of the height (distance) between the tips of the small needles of the microneedle and the tip surfaces of the fixing members is equal to "the shortest distance between four rectangular columns" $\times$ 0 to 16%.
(8) The microneedle device according to (5), wherein tip portions of small needles of the microneedle are positioned within the device and less than tip surfaces of the skin fixing members, and the difference of the height (distance) between the tips of the small needles of the microneedle and the tip surfaces of the fixing members is equal to "the shortest distance between three rectangular columns" $\times$ 0 to 30%.
(9) The microneedle device according to any of (1) to (8), wherein the flat plate has a square, rectangular or circular outline.
(10) The microneedle device according to (9), wherein the peripheral portion of the flat plate represents corners of a square or a rectangle.

(11) A microneedle device with a microneedle placed on a flat plate, **characterized in that**
the device comprised two or more skin fixing members, for fixing the skin, at a peripheral portion of the flat plate,
a fixed area of the skin defined by the fixing members is 5 $\times$ 5 mm to 70 $\times$ 70 mm,
the fixing members each have a prismatic column shape, circular column shape, barrel-roof shape (semicircular column shape), or hemispherical shape,
the microneedle is placed on the flat plate at a central portion between/among the fixing members, and
the difference of the height (distance) between tip portions of the microneedle and tip portions of the fixing members is equal to "the height of elevation of the skin during the pressing" - "the length of small needles".
(12) The microneedle device according to (11), wherein the height of elevation of the skin during the pressing is equal to "the shortest distance between fixing members" $\times$ 16%.
(13) The microneedle device according to (11) or (12), wherein the flat plate has a square shape, rectangular shape, or circular shape.
(14) The microneedle device according to any of (1) to (13), wherein a protrusion for pressing is placed on the central portion of the flat plate.
(15) The microneedle device according to (14), wherein the protrusion has a circular column-shape or hemispherical shape.
(16) The microneedle device according to any of (1) to (13), wherein an arched structure is placed on a top portion of the flat plate such that force is applied to the fixing members uniformly.

(17) A tool for measuring the angle of elevation of the skin surface , **characterized in that**

a) a circular column or a prismatic column is perpendicularly placed on a central portion of a circular disk to be placed on the skin surface,

b) the device according to (1) to (13) is used, in which the microneedle is not present and an opening through which said circular column or said prismatic column can be passed is made in the central portion of the device, and

c) the circular column or the prismatic column is fit in the opening.

(18) A method of measuring the angle of elevation of the skin surface, comprising:

using the tool for measuring the angle of elevation of the skin according to (17), and

measuring the angle between the column and the device surface during the pressing of the skin, by means of a protractor.

(19) The microneedle device according to (1) to (16), wherein a tape-like chip to be adhered to the skin is placed.

(20) The microneedle device according to claim 12, wherein the tape-like chip has the following characteristics:

a) the tape-like chip is of 1 to 50 cm$^2$,

b) a support of the tape-like chip and the device are adhered together with an adhesive, and

c) a microneedle of 5 to 15 mm$\phi$ is placed on a central portion of the tape-like chip.

(21) The microneedle device according to (19) or (20), wherein the adhesion of the adhesive is 85 to 90% or less of that of an adhesive for adhering to the skin.

(22) The microneedle device according to (19) or (20), wherein the difference between the adhesion of the adhesive for adhering the support to the device and the adhesion of the adhesive for adhering to the skin is 10% or more of the adhesion of the adhesive for adhering to the skin.

(23) The microneedle device according to any of (19) to (22), wherein the adhesive for adhering the support to the device and the adhesive for adhering to the skin are made of the same material, and the area of the adhesive for adhering to the device is 85 to 90% or less of that of the adhesive for adhering to the skin.

(24) The microneedle device according to any of (19) to (23), wherein the shape of the area of the adhesive for adhering the support to the device is selected from the group consisting of star shape, rhombus shape, triangle shape, quadrangle shape, cross shape, wavy line shape, circular shape, annular shape, and ellipse shape, and the adhering is carried out with one or more adhesive(s).

(25) The microneedle device according to any of (19) to (24), wherein the tape-like chip is a circular shape or a rectangle shape.

(26) The microneedle device according to any of (19) to (25), wherein the adhesive for adhering the support to the device is placed on the inner side from the periphery of the tape-like chip.

(27) The microneedle device according to (26), wherein the state in which the adhesive is placed on the inner side from the periphery of the tape-like chip means a state in which the adhesive is placed at a position 0.1 mm or more away from the periphery of the tape-like chip.

**EFFECTS OF THE INVENTION**

The device of the present invention is, when pressed against the skin, regardless of the position of the flat plate of the device where force is applied, capable of elevating the skin surface in the space between/among the skin fixing members while keeping the skin surface parallel to the flat plate of the device. This makes it possible to insert the microneedle placed which is on the flat plate surface of the device, into the skin surface perpendicularly. Therefore, the device enables skin puncture, hardly causing breakage of the small needles, even though the microneedle is made of a biodegradable resin that has insufficient material strength. Further, since the device of the present invention is in the form of a small chip, it is easily portable, with the concave part being covered, and even ordinary people can remove the cover and administer a drug transdermally in a convenient manner.

Further, a patch (a tape-like chip) for retaining a microneedle, which is suitable for the device of the present invention, has been developed. That is, there is manufactured a tape-like chip, wherein adhesives are applied to both faces of a support of the tape-like chip, and the adhesion of the adhesive at the device side is set to be 85 to 90% or less of that of the adhesive for the skin. With the device of the present invention, the tape-like chip makes it possible to puncture the skin with the microneedle conveniently and retain the microneedle therein easily. The device of the present invention, in which the tape-like chip including the microneedle is held, has an extremely simple structure. In addition, by adjusting the adhesion of the adhesive, missing of the tape-like chip including the microneedle from the applicator does not occur during transportation. Thus, there is provided an applicator which is easily portable and allowing for puncturing the skin in a simple way, thereby achieving administration with a microneedle by easy self-administration regarding drugs which had to be administered subcutaneously, for example.

BRIEF DESCRIPTION OF THE DRAWINGS

Fig. 1 shows schematic diagrams of three types of applicators of the present invention.
Fig. 2 shows drawings showing aspects of how to place a clog-shaped applicator of the present invention with respect to the arm (perpendicular placement and parallel placement).
Fig. 3 is a diagram showing a correlation between the distance b and the height (distance) of elevation of the skin, when a clog-shaped applicator of the present invention is provided in the perpendicular placement with respect to the arm and pressed against it.
Fig. 4 is a diagram showing a correlation between the distance b and the height (distance) of elevation of the skin, when a clog-shaped applicator of the present invention is provided in the parallel placement with respect to the arm and pressed against it.

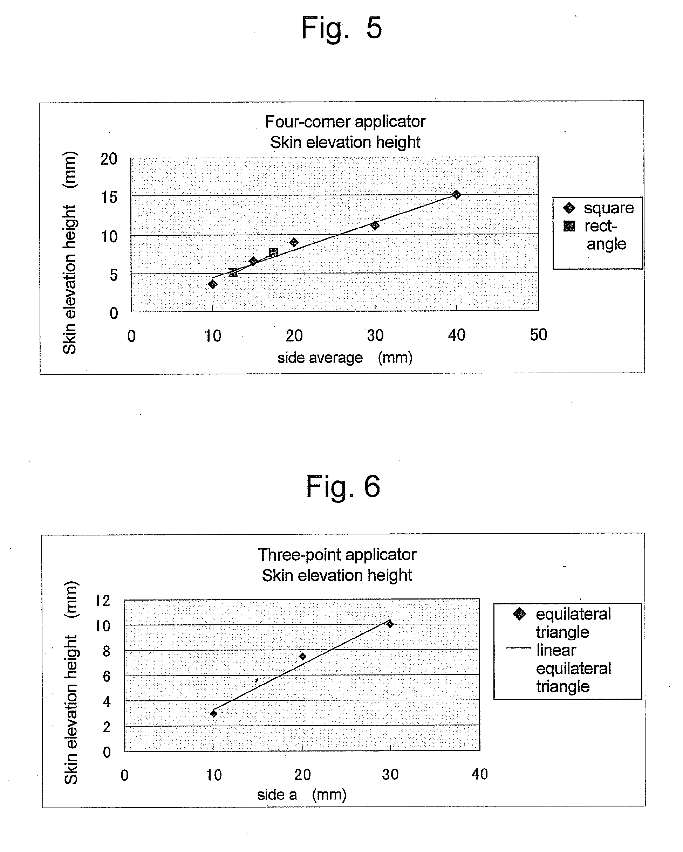

Fig. 5 is a diagram showing a correlation between the average of the distance a and the distance b and the height (distance) of elevation of the skin, when a four-corner applicator of the present invention is placed on the arm and pressed against it.
Fig. 6 is a diagram showing a correlation between the distance a and the height (distance) of elevation of the skin, when a three-point applicator of the present invention is placed on the arm and pressed against it.

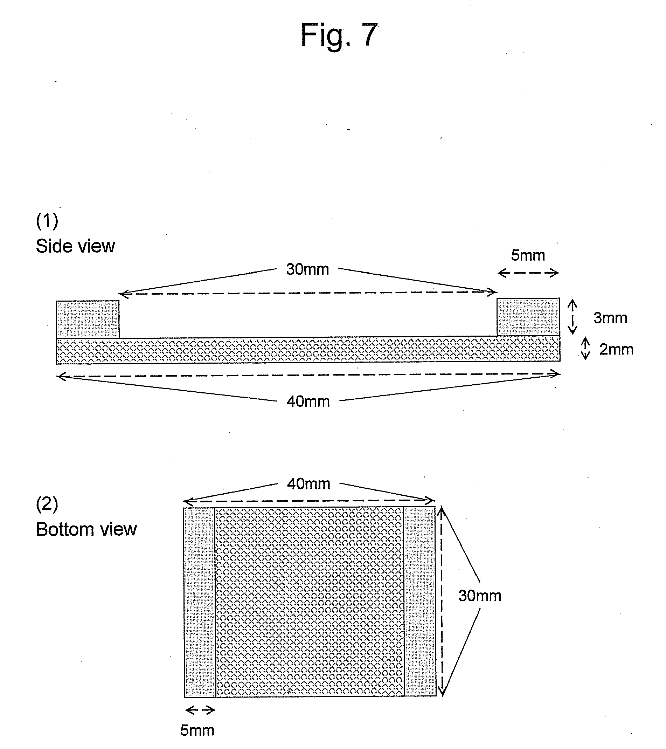

Fig. 7 shows drawings showing an example of a clog-shaped applicator of the present invention.

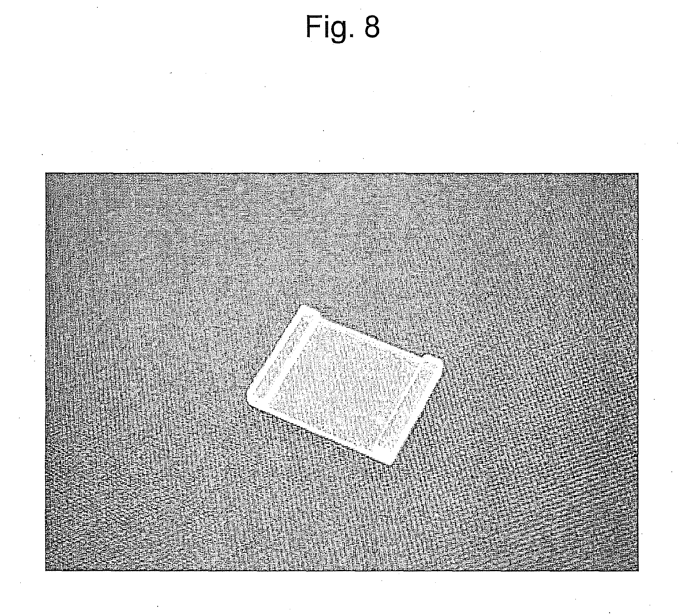

Fig. 8 is a perspective view of a clog-shaped applicator made of polypropylene, which is manufactured based on the drawings of Fig. 7.

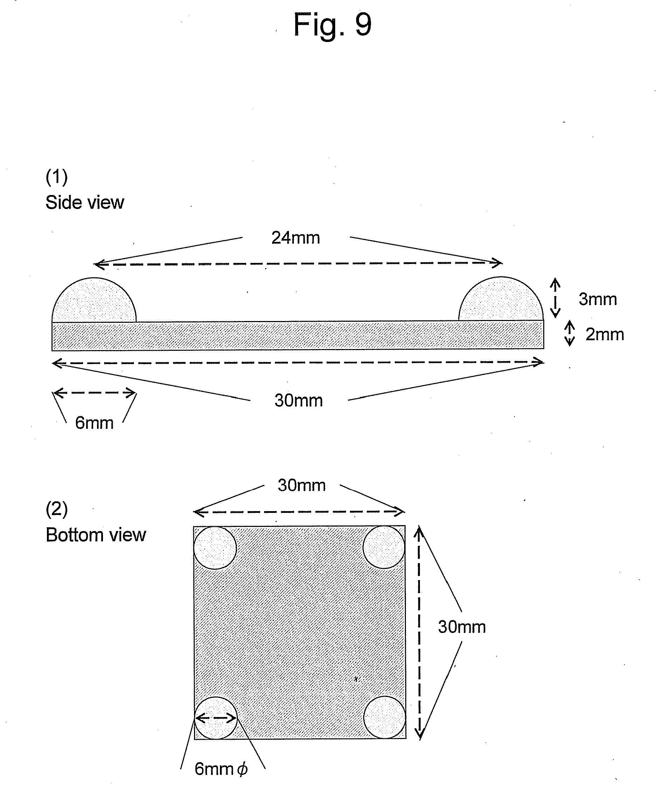

Fig. 9 shows drawings showing an example of a four-corner applicator having hemispherical-shaped skin fixing members.

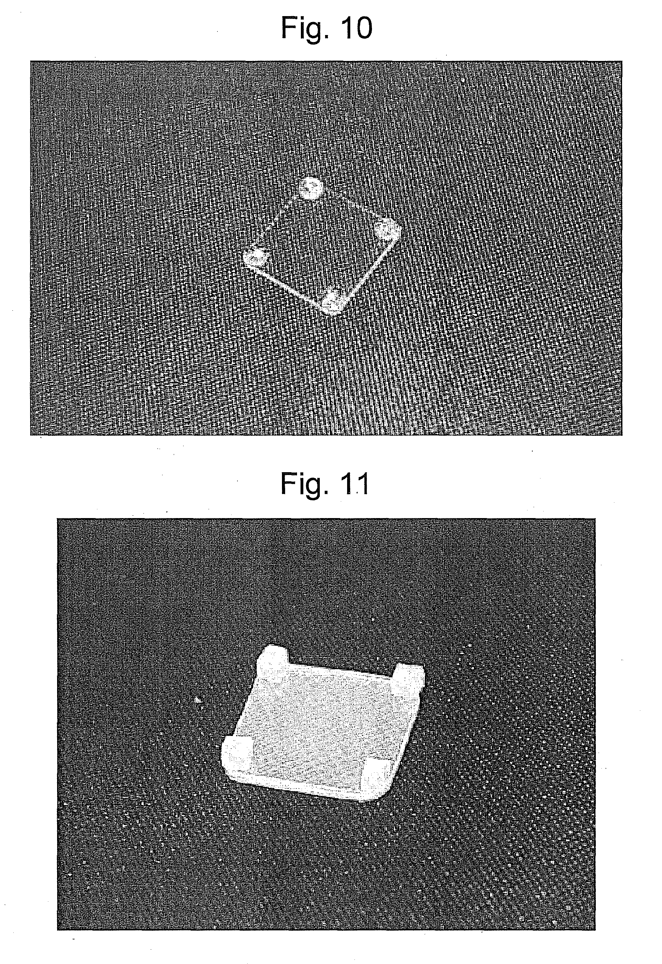

Fig. 10 is a perspective view of a four-corner applicator made of polypropylene, which is manufactured based on the drawings of Fig. 9.
Fig. 11 is a perspective view of a four-corner applicator made of polypropylene, which is manufactured based on the drawing of Fig. 1(2).
Fig. 12 is a perspective view of a three-point applicator made of polypropylene, which is manufactured based on the drawing of Fig. 1(3).
Fig. 13 is a side view (photograph) showing a state in which a clog-shaped applicator of the present invention is provided in the perpendicular placement with respect to the arm and pressed against it so that the skin is elevated.

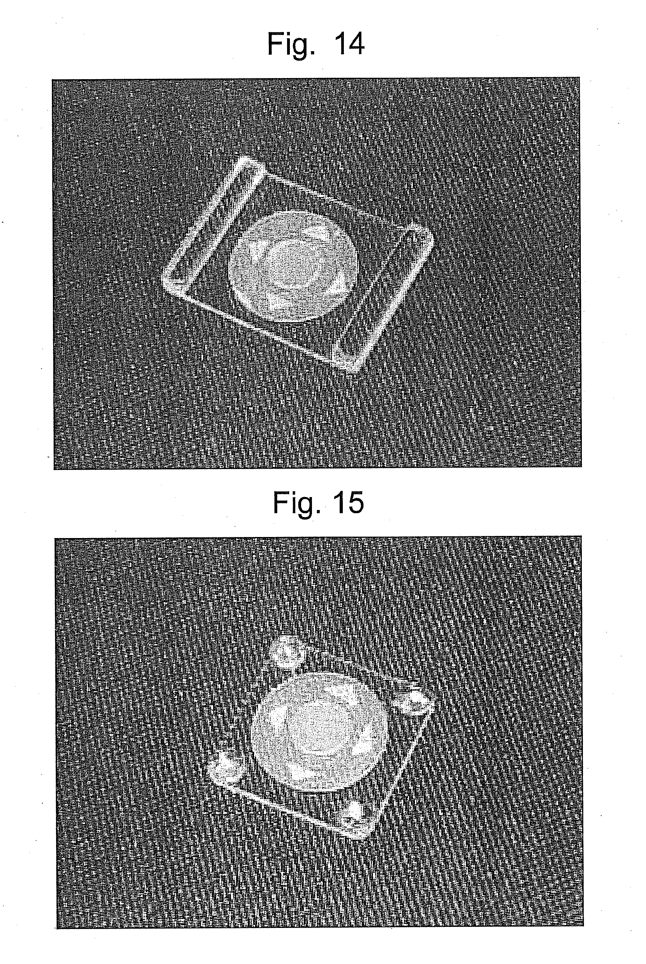

Fig. 14 is a perspective view of a clog-shaped applicator (device) of the present invention, prepared by placing triangular double-sided tapes on the clog-shaped applicator of Fig. 8 at four places, overlapping a circular adhesive tape thereon (adhering of the double-sided tapes and the support), and further placing a circular microneedle on the circular adhesive tape.
Fig. 15 is a perspective view of a four-corner applicator (device) of the present invention, prepared by placing triangular double-sided tapes on the four-corner applicator of Fig. 10 at four places, overlapping a circular adhesive tape thereon (adhering of the double-sided tapes and the support), and further placing a circular microneedle on the circular adhesive tape.

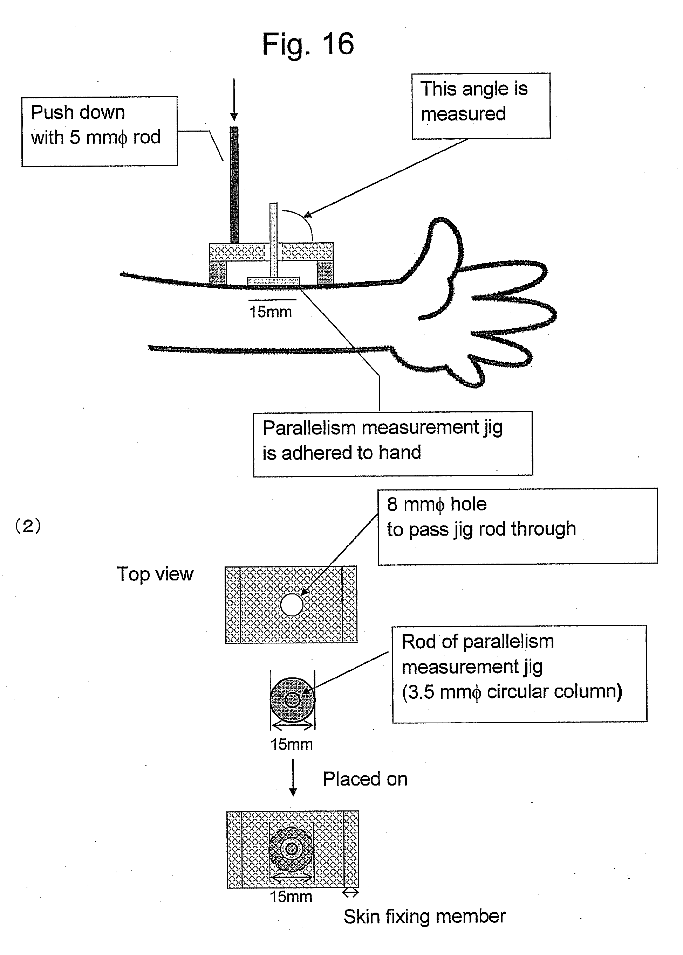

Fig. 16 shows schematic diagrams showing the structure of a parallelism measuring jig for elevation of the skin.

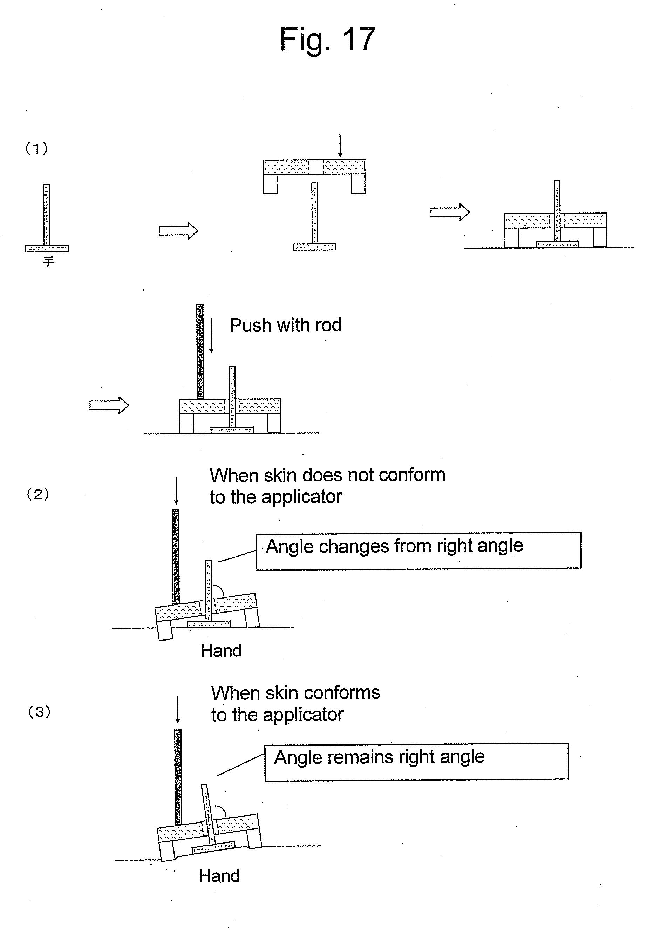

Fig. 17 shows drawings showing that, when the parallelism measuring jig of Fig. 16 is used and each place of the flat plate of the applicator (places shifted from the center) is pressed, the movement of the skin is always coordinated with the movement of the applicator.

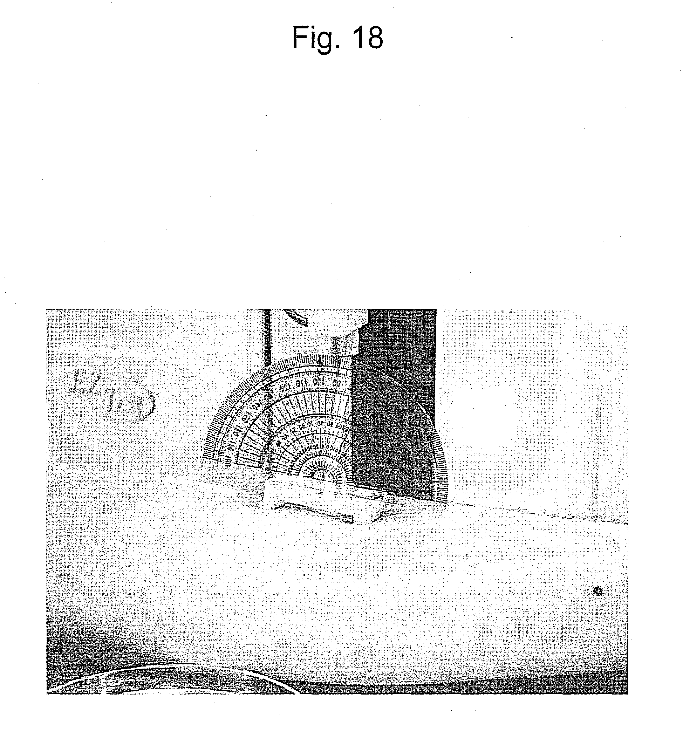

Fig. 18 is a drawing (photograph) showing a state in which, regarding a clog-shaped applicator, the parallelism measuring jig of Fig. 16 is used and pressed in the same manner as in Fig. 17. A protractor shows that the skin is coordinated with the applicator and the skin surface rises perpendicularly as shown in Fig. 17(3).

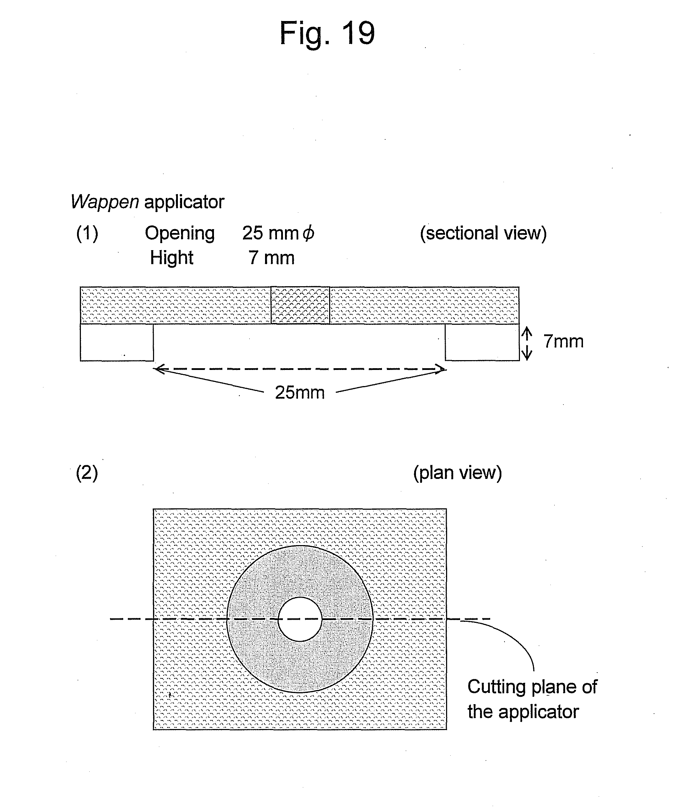

Fig. 19 shows drawings of (1) a cross-sectional view and (2) a plan view of a *wappen*-shaped applicator to be used with the parallelism measuring jig of Fig. 16.
Fig. 20 is a pattern diagram schematically showing a side view of the clog-shaped applicator of Fig. 14.
Fig. 21 is a schematic diagram showing an assessment method of skin puncture properties.

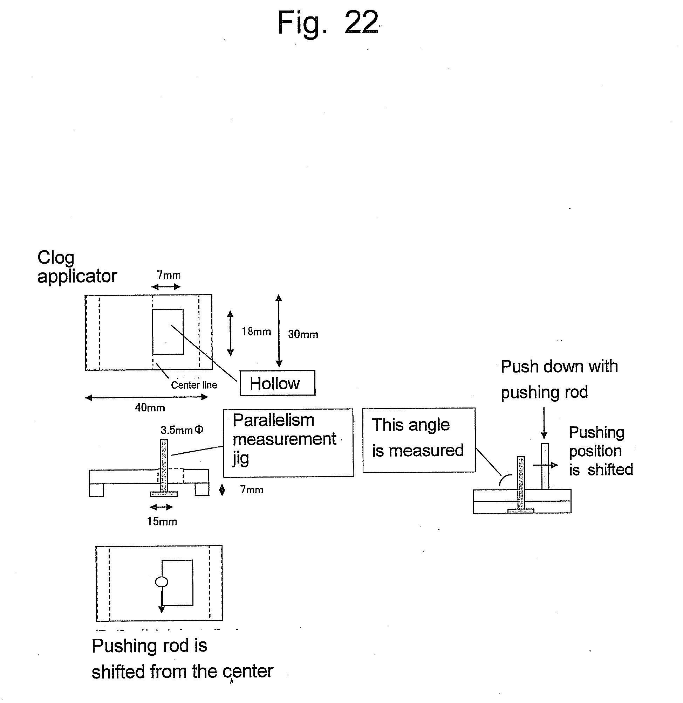

Fig. 22 shows drawings showing a state in which, regarding a clog-shaped applicator, when the pressing site is shifted from a central portion of a top plate in a direction where there is no skin fixing member, the movement of the skin (how the skin rises perpendicularly) is assessed by means of the parallelism measuring jig in the same manner as in Fig. 18.

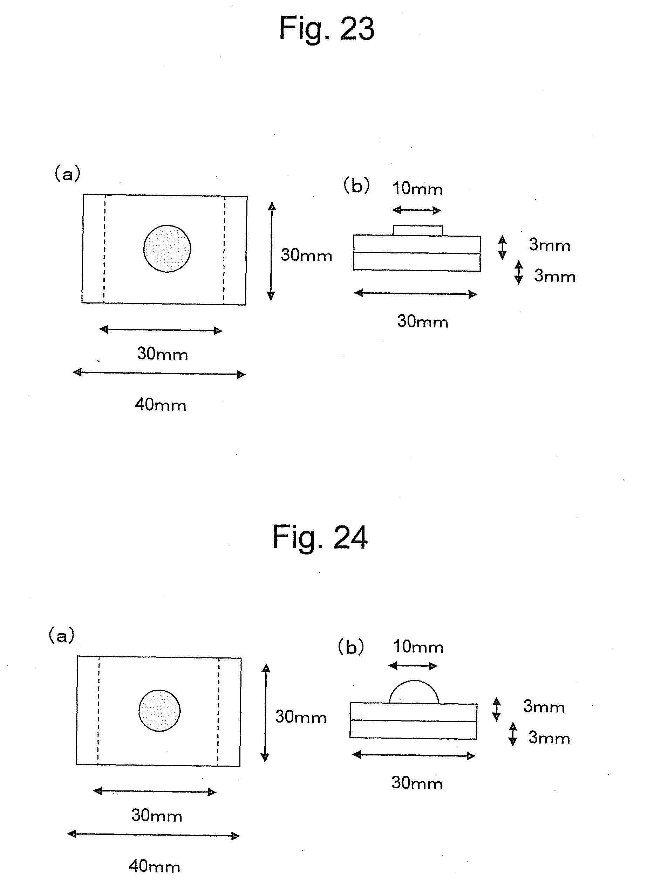

Fig. 23 shows drawings of a front view and a side view of a microneedle device (applicator), wherein a circular column shaped pressing part is placed on a central portion of a top plate of a clog-shaped applicator.

Fig. 24 shows drawings of a front view and a side view of a microneedle device (applicator), wherein a hemispherical-shaped pressing part is placed on a central portion of a top plate of a clog-shaped applicator.

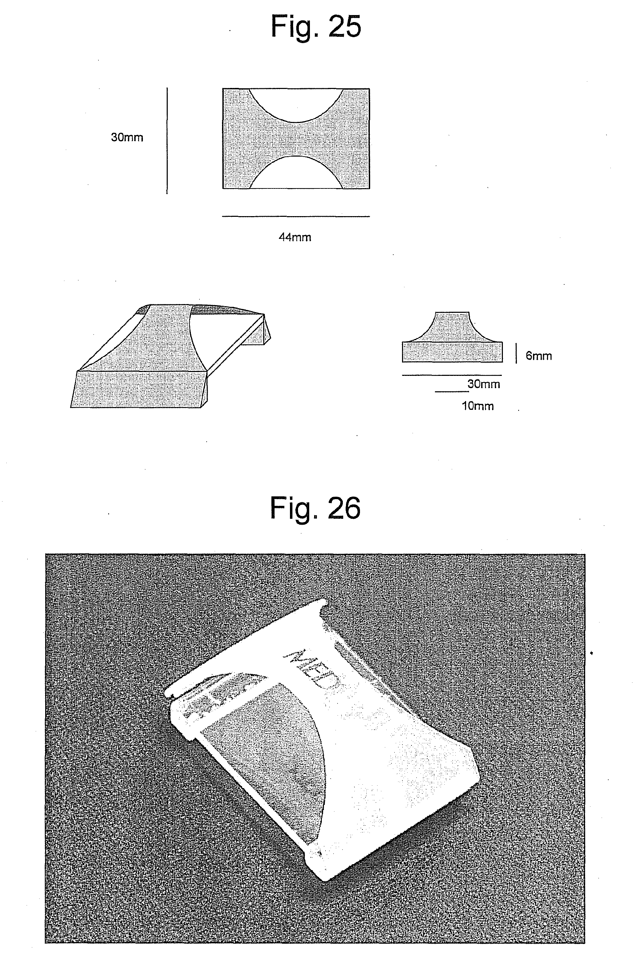

Fig. 25 shows drawings of a front view, a side view, and a perspective view of a microneedle device (applicator), wherein an arched structure is placed on a top plate of a clog-shaped applicator.

Fig. 26 is a perspective view (photograph) of the microneedle device (applicator), wherein an arched structure is placed on a top plate of a clog-shaped applicator.

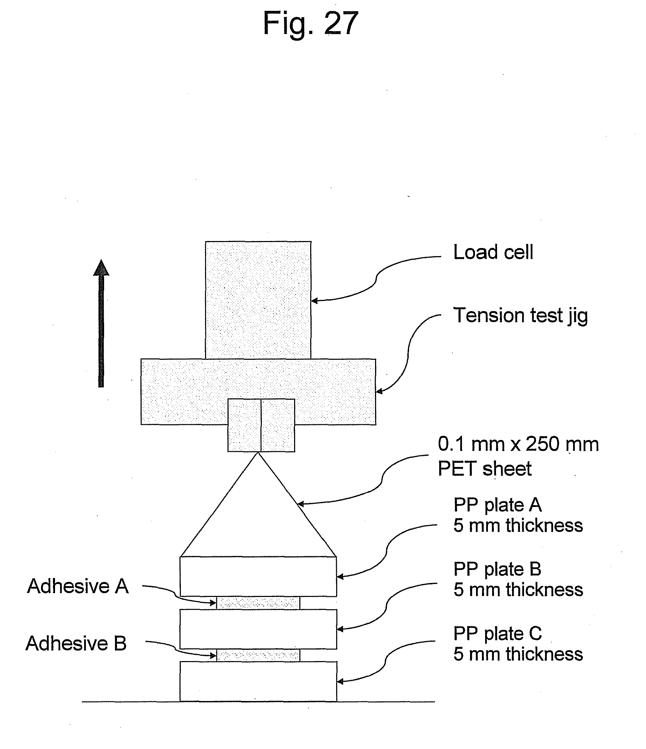

Fig. 27 is a schematic diagram of a test method for assessing vertical tensile stress of adhesives applied to both faces of a tape-like chip of the present invention (measurement of tensile stress of the adhesion of the adhesives to a polypropylene flat plate).

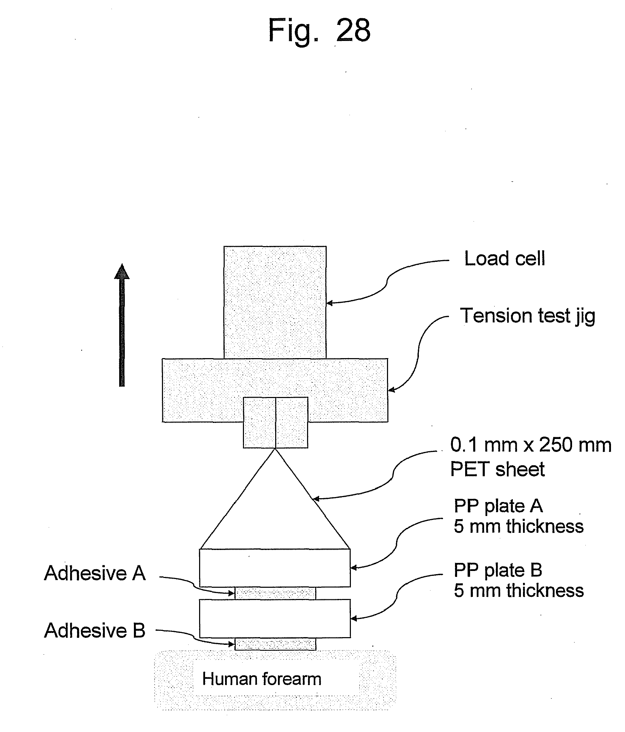

Fig. 28 is a drawing showing a measurement result of a schematic diagram of a test method for assessing vertical tensile stress of adhesives applied to both faces of a tape-like chip of the present invention (tensile stress of the adhesion of the adhesives to the skin (a polypropylene flat plate and the skin)).

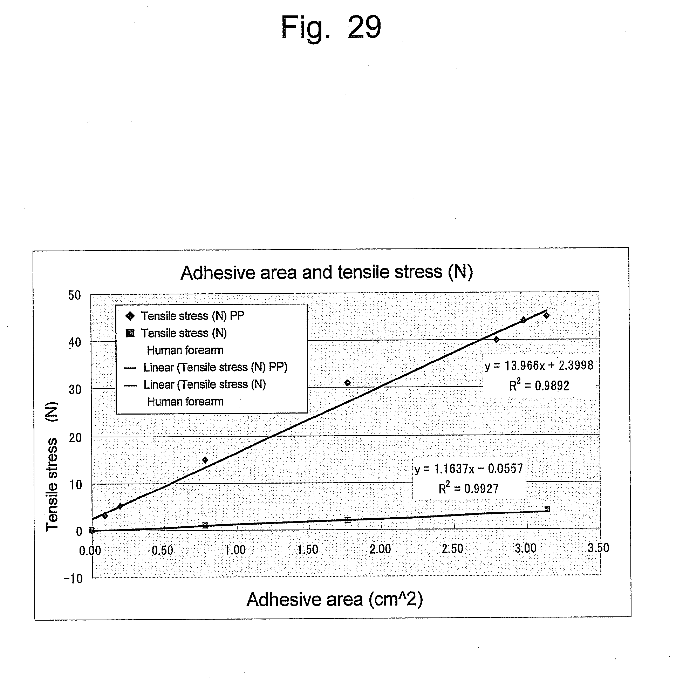

Fig. 29 shows (measurement of) tensile stress of the adhesion of adhesives to the skin.

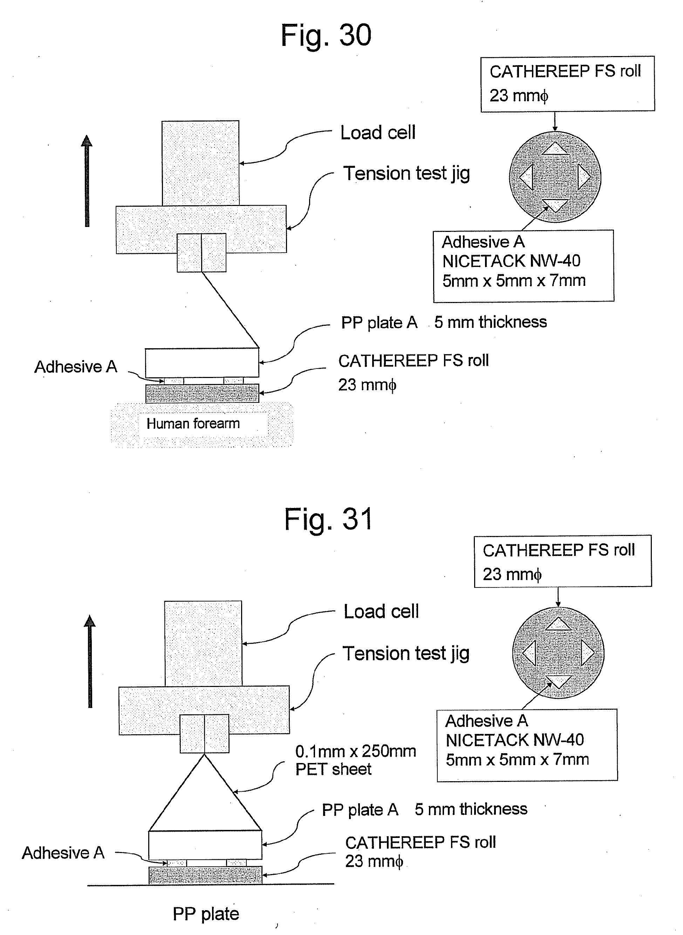

Fig. 30 is a schematic diagram of a test method for assessing diagonal tensile stress of an adhesive regarding a tape-like chip of the present invention (measurement of tensile stress of the adhesion of the adhesive to the skin).

Fig. 31 is a schematic diagram of a test method for assessing vertical tensile stress of an adhesive regarding the tape-like chip used in Fig. 30 (measurement of tensile stress of the adhesion of the adhesive to a polypropylene flat plate).

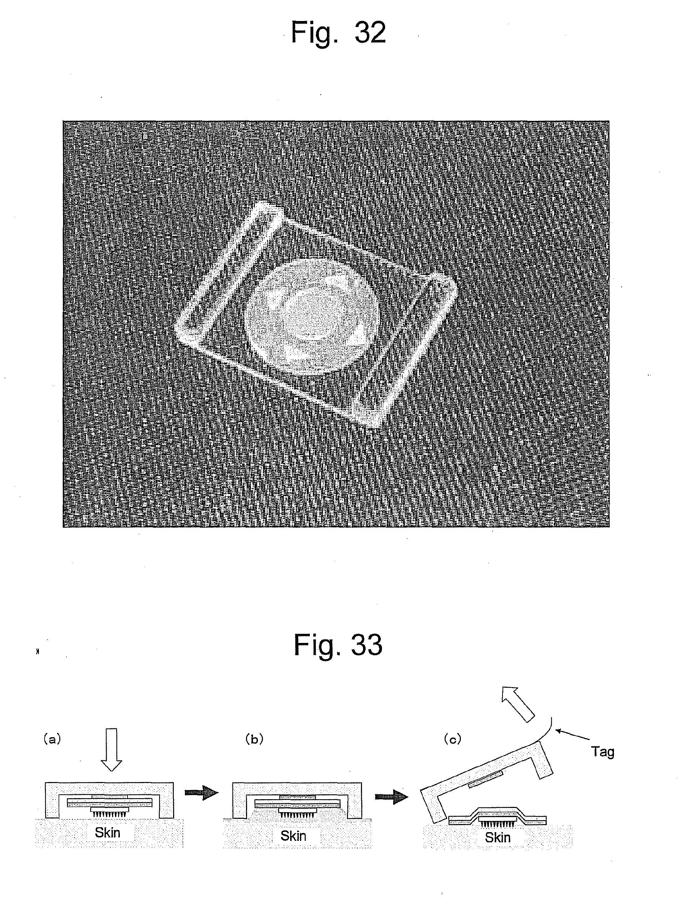

Fig. 32 is a perspective view (photograph) of a clog-shaped applicator (made of polypropylene) to which a tape-like chip including a microneedle is adhered.

Fig. 33 shows concept views showing a series of procedures including steps of placing a device (applicator) of the present invention on the skin, pressing it against the skin, allowing a tape-like chip to be firmly attached to the skin, thereafter pulling up the device, and retaining the tape-like chip in the skin.

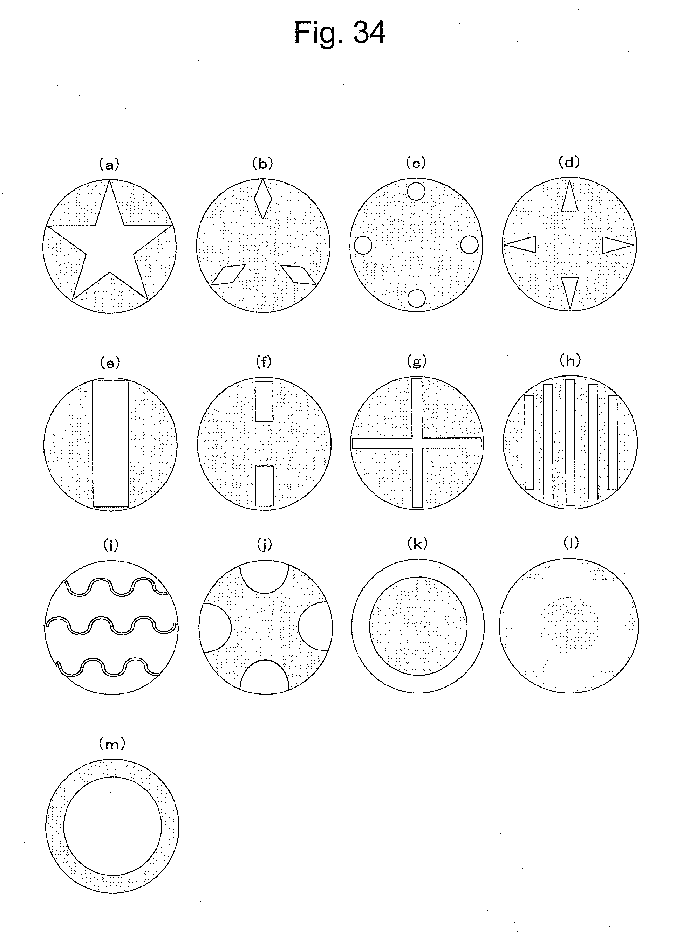

Fig. 34 shows drawings showing application patterns of an adhesive at the applicator (device) side of a tape-like chip (circle), wherein white color indicates the portion to which the adhesive is applied.

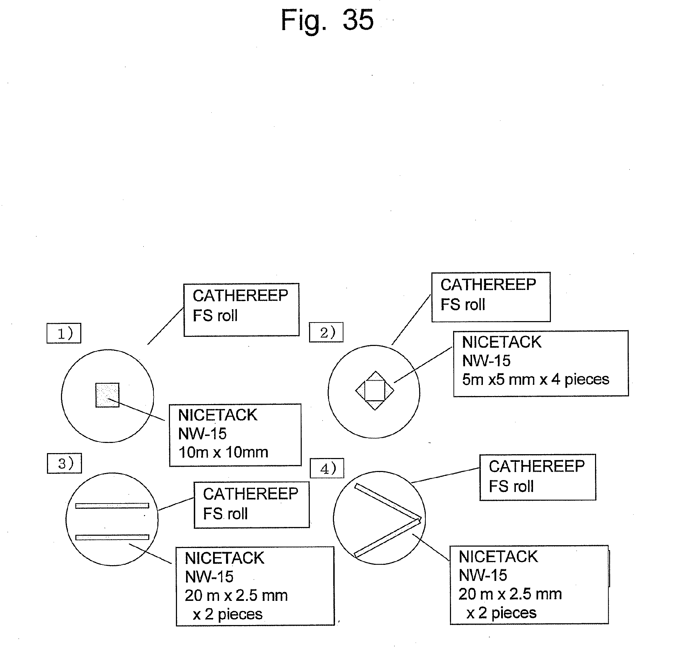

Fig. 35 shows drawings showing that, application areas of an adhesive at the applicator (device) side of a tape-like chip (circle) are the same; however, application patterns of the adhesive are different in shape, wherein gray color indicates the portion to which the adhesive is applied.

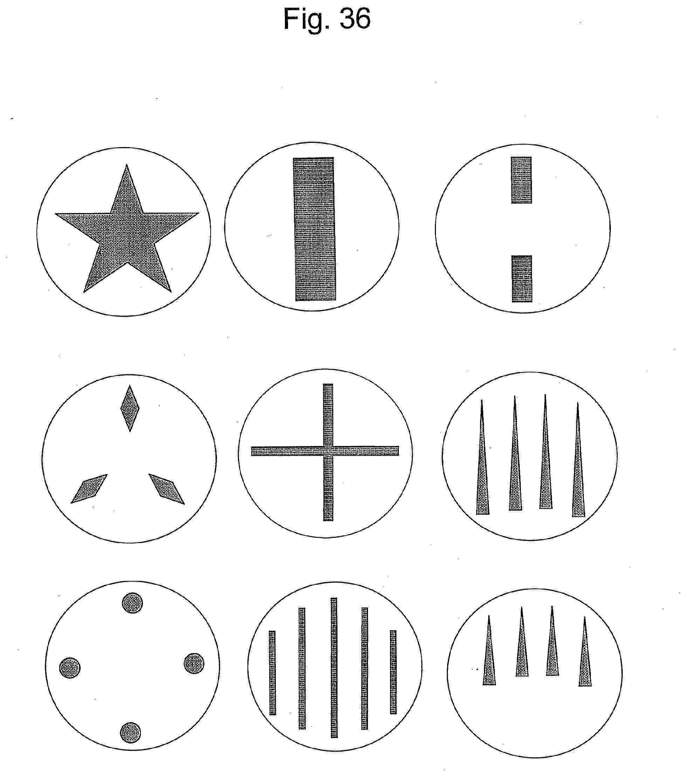

Fig. 36 shows drawings showing application patterns of an adhesive at the applicator side of a tape-like chip (circle), wherein gray color indicates the portion to which the adhesive is applied.

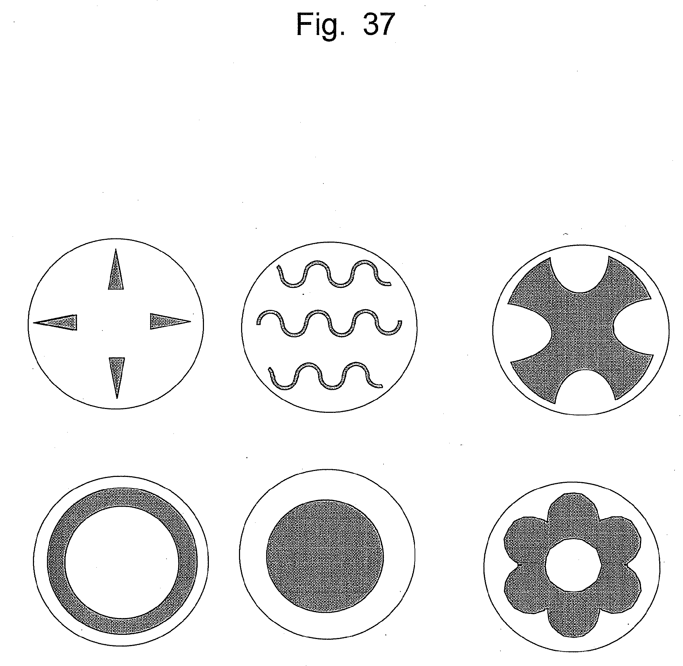

Fig. 37 shows drawings showing application patterns of an adhesive at the applicator side of a tape-like chip (circle), wherein gray color indicates the portion to which the adhesive is applied.

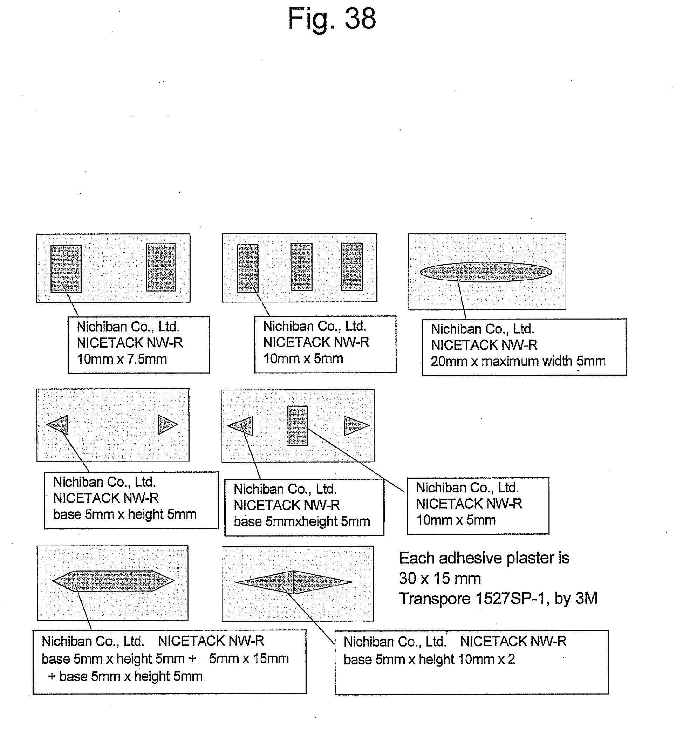

Fig. 38 shows drawings showing application patterns of an adhesive at the applicator side of a tape-like chip (rectangle), wherein gray color indicates the portion to which the adhesive is applied.

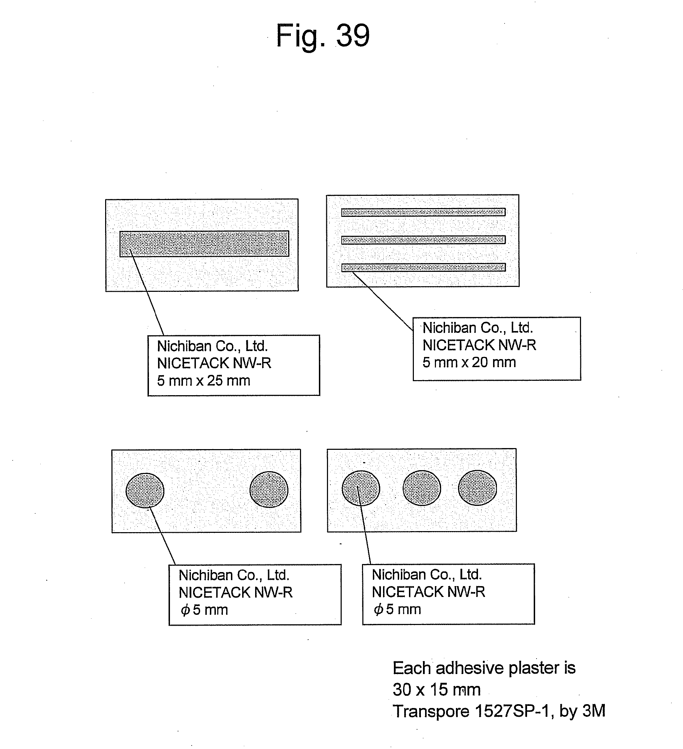

Fig. 39 shows drawings showing application patterns of an adhesive at the applicator side of a tape-like chip (rectangle), wherein gray color indicates the portion to which the adhesive is applied.

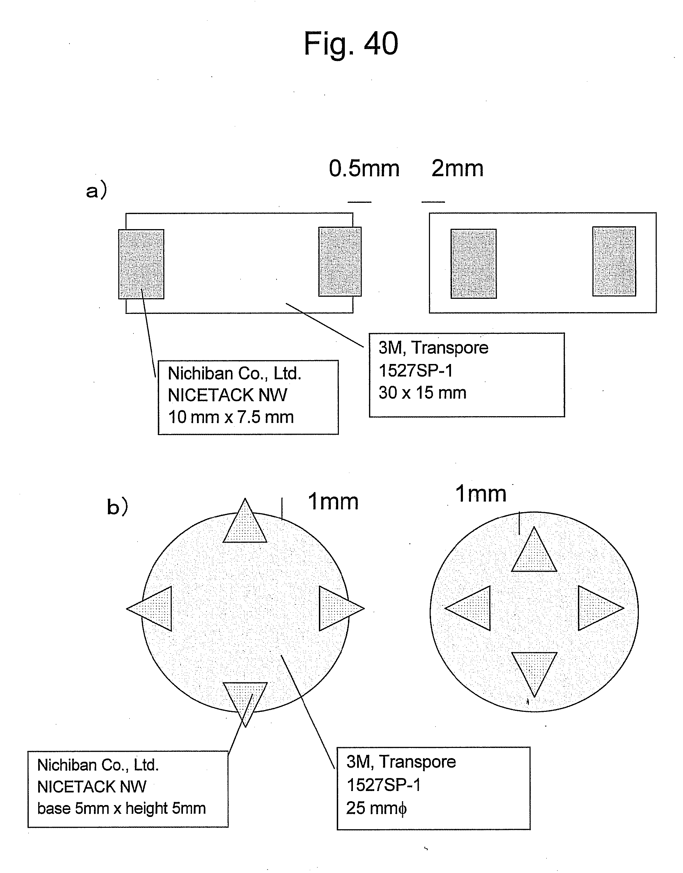

Fig. 40 shows drawings showing application patterns of an adhesive at the applicator side of tape-like chips (circle and rectangle), wherein gray color indicates the portion to which the adhesive is applied. Here, the adhesive is applied to a position away from the periphery of the tape-like chips (a protruded position or inner position from the periphery).

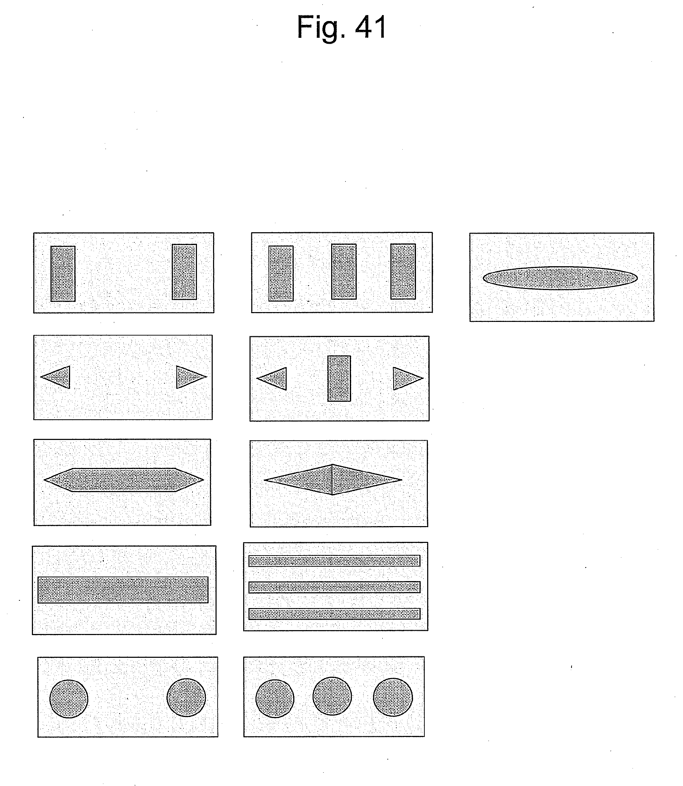

Fig. 41 shows drawings showing application patterns of an adhesive at the applicator side of a tape-like chip (rectangle), wherein dark gray color indicates the portion to which the adhesive is applied.

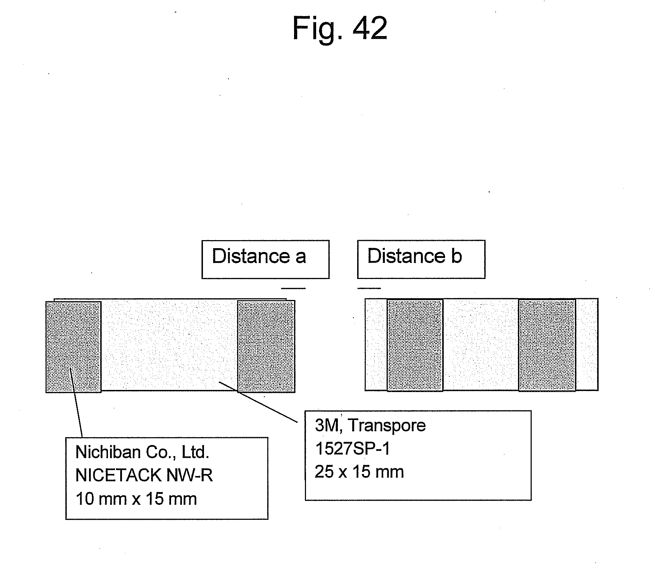

Fig. 42 shows drawings of a tape-like chip prepared in order to confirm that the property of transfer of the chip to the skin is changed depending on the position of an adhesive used in a tape-like chip (rectangle) (the distance from the periphery of the tape-like chip).

## EMBODIMENTS FOR CARRYING OUT THE INVENTION

Hereinafter, the present invention will be further explained in detail with reference to preferable aspects shown in the appended drawings.

An applicator device of the present invention is, for example, the one shown in Fig. 14 or 15. The device is a chip-shaped tool (e.g. clog-shaped applicator), wherein a microneedle is fixed to an adhesive tape for the skin, and a support (backing) of the adhesive tape is fixed to a flat plate of the device with a weaker adhesive member such as a double-sided tape, as shown in Fig. 20.

Here, by applying the weaker adhesive on the surface of skin fixing members and allowing a cover film to be adhered thereto, it is possible to use this cover film as a protective sheet of small needles of the microneedle and prevent the breakage of the small needles.

To use the chip-shaped tool of the present invention, first the cover sheet is detached therefrom, and then the chip-shaped tool is placed on the skin surface.

When the microneedle device of the present invention, particularly a clog-shaped applicator of Fig. 1(1), is used, the height of elevation of the skin surface differs depending on the direction of placement of the applicator as shown in Fig. 2 (see Figs. 3 and 4); however, when the devices of Figs. 1(2) and (3) are used, the height of elevation of the skin is not affected by the direction of placement of the devices (see Figs. 5 and 6).

When the clog-shaped applicator is used, for example, it is provided in perpendicular placement with respect to the direction of the arm as shown in Fig. 13 and pressed down about 12 mm from the placed position. Then, the skin is elevated in the space defined between skin fixing members (space defined by the distance a and the distance b). The height of the elevated skin (the distance between the skin fixed by the skin fixing members and the peak of the elevated skin) is measured with a caliper by using a hole of 8 mm$\phi$ opened for the purpose of measurement at the center of a flat plate of the applicator.

The flat plate member of the applicator device is preferably a hard member. Examples of the hard member include those made of various materials, for example, synthetic resins, metals, wood, and the like, with at least a certain thickness. Examples of the synthetic resins include hard resins such as polyethylene, polypropylene, polyvinyl chloride, acrylic, polyethylene terephthalate, polystyrene, acrylonitrile-butadiene-styrene copolymer, polycarbonate, polyamide, fluororesin, and polybutylene terephthalate.

The skin fixing member is not particularly limited to any material. In terms of processability, it may be preferably made of resins such as polyethylene, polypropylene, polyvinyl chloride, acrylic, polyethylene terephthalate, polystyrene, acrylonitrile-butadiene-styrene copolymer, polycarbonate, polyamide, fluororesin, and polybutylene terephthalate.

The location of the skin fixing members is preferably selected such that the skin is fixed at an approximately equal distance from the microneedle placed at the center. Therefore, it is preferred to locate the skin fixing members point-symmetrically with the microneedle being at the center, or locate the fixing members at corners of a regular polygon with the microneedle being at the center. For example, when two skin fixing members are located, rectangular column shaped or semicircular column shaped members may be located in a clog-shaped configuration. When three to seven skin fixing members are located, they may be located at apexes of a regular polygon. The shape of the skin fixing member may be rectangular column shape, circular column shape, or hemispherical shape.

A protrusion (structure) for pressing, which is placed on the flat plate, preferably has a size suitable for the pressing with a finger, and the shape thereof is not particularly limited. Preferably, the protrusion may be a circular column-shaped or hemispherical-shaped protrusion as shown in Figs. 23 and 24, or may be an arched structure as shown in Figs. 25 and 26. The material of the member for pressing may be the same as that of the flat plate member or that of the skin fixing members. Further, these members (the member for pressing, the flat plate member, and the skin fixing member) may be formed integrally by means of injection molding or the like.

The position where the protrusion (structure) for pressing is placed is preferably the central portion of the device where the microneedle is placed. That is, it is preferably the same position as that of the microneedle but a top portion of the flat plate of the device. When the protrusion is an arched structure, the position to be pressed with a finger is preferably set to be at the central portion of the device.

Here, when the protrusion (structure) for pressing is not placed on the flat plate of the device of the present invention, it is preferred to provide a print or seal on the flat plate in order to clearly show the position to be pressed. Further, it is also preferred to make a slight dent in the position to be pressed on the flat plate in order to clearly show the position to be pressed.

The size of the applicator device of the present invention is affected by the size (diameter or long side) of the substrate (base for small needles) of the microneedle to be used.

For example, as shown in Figs. 14 and 15, when the diameter of the base of the microneedle is about 1 cm, the diameter of a packing portion is preferably approximately 2.5 mm. Therefore, in the applicator device of the present invention, the distance between the skin fixing members (distance b) is preferably approximately 3 cm, and the length of the skin fixing members (distance a) is preferably approximately 3 cm.

On the other hand, when the size of the applicator device is fixed, the size of the base of the microneedle is accordingly decided.

In the applicator device of the present invention, the microneedle can be protected by a cover film adhered between the skin fixing members.

Since there is the cover film, the small needles of the microneedle are protected against accidental contact with outside, and this makes it easy to carry the device of the present invention. After the cover film is detached from the device, the device of the present invention can be placed on the skin surface conveniently and puncture the skin with the microneedle.

When the applicator device of the present invention is pressed against the skin, the skin is elevated to the position of the microneedle. This is affected by the skin fixing members of the present invention and location configuration thereof. When applicators having the shapes shown in Fig. 1 are used, the height of elevation of the skin surface is affected depending on the location configuration of the skin fixing members, as shown in Figs. 3 to 6. The height of elevation of the skin surface is also affected by the way how the applicator device is placed with respect to the arm. Preferable examples of the placement of the applicator device include perpendicular placement with respect to the direction of the arm as shown in Fig. 13.

As shown in Figs. 3 to 6, the height of elevation of the skin depends on the area defined between/among the skin fixing members (area defined by the distance a and the distance b). Therefore, the distance between the skin fixing members is preferably 5 to 70 mm, and more preferably 10 to 40 mm. Further, the area defined between/among the skin fixing members (area defined by the distance a and the distance b) is preferably $5 \times 5$ mm to $70 \times 70$ mm, and more preferably $10 \times 10$ mm to $40 \times 40$ mm.

Examples of the shape of the flat plate of the applicator of the present invention include a circle, an ellipse, quadrangles such as a square and a rectangle, and polygons such as a pentagon and a hexagon. More preferable examples of the shape include a circle, a square, and a rectangle. The size of the flat plate of the applicator of the present invention is mainly defined by the distance between the skin fixing members of 5 to 70 mm, and is affected by the width of the skin fixing members. For example, when the width of the skin fixing members is 5 mm as shown in Fig. 7, the flat plate may be a square or a rectangle having a side length of 25 to 60 mm, or preferably a square or a rectangle having a side length of 25 to 40 mm.

As for the size of the skin fixing members, in the case of rectangular columns, the side length thereof is 5 to 7 mm; and in the case of hemispheres, the diameter thereof is 5 to 7 mm$\phi$.

The height of elevation of the skin is the distance between the skin fixed by the skin fixing members and the peak of the elevated skin. Figs. 3 to 6 show that the height of elevation of the skin (distance) has a positive correlation with the distance b or the average of distances a and b. Accordingly, by adjusting the height of the skin fixing members placed on the flat plate of the applicator appropriately, tips of small needles of the microneedle approach the skin. Thus, by pressing the applicator only a short distance, the microneedle is inserted into the skin easily. In consideration of the heights of the small needles and the base of the microneedle, the thickness of the adhesive tape, etc., the height of the skin fixing members is preferably about 1 to 15 mm, more preferably about 1 to 7 mm.

The tips of the microneedle placed on the applicator device of the present invention are positioned in a range of preferably 0 to 5 mm, more preferably 0 to 3 mm, from the tip surfaces of the skin fixing members.

The microneedle (pin-frog-shaped) of the present invention may be a publicly-known microneedle made of various materials for various uses. Examples thereof include a microneedle made of silicone, such as silicone and ceramics; those made of metals, such as stainless steel, tungsten steel, nickel alloy, molybdenum, chrome, cobalt, titanium and alloy thereof; a microneedle made of biodegradable resins including aliphatic polyester resins such as polylactic acid, polyglycolic acid, a copolymer of polylactic acid and polyglycolic acid; and a microneedle made of polysaccharides such as maltose, lactose, sucrose, mannitol, and sorbitol. Among them, a microneedle made of aliphatic polyester resins, which is biodegradable materials, or those made of polysaccharides have relatively low strength and tend to be weak to impact and easily broken; however, the use of the applicator of the present invention makes it possible to prevent or reduce the breakage of the tip needle portions of the microneedle and puncture the skin surface with the microneedle surely.

Thus, it has been made possible to market, as a product, microneedles made of biodegradable resins typified by aliphatic polyester resins, such as polylactic acid, polyglycolic acid, a copolymer of lactic acid and glycolic acid, or microneedles made of polysaccharides, such as maltose, lactose, sucrose, mannitol, and sorbitol, all of which have been difficult to use effectively.

The entire shape of the microneedle (pin-frog-shaped) is preferably a circle, and the size thereof may be 5 to 15 mm$\phi$, and preferably 9 to 13 mm$\phi$.

Also, in the method of the present invention, it is possible to puncture the skin surface with the microneedle and then easily retain the microneedle on the skin, thereby enabling an improved absorption or a sustained-release property of a drug by using the microneedle made of a biodegradable material.

The adhesive tape of the present invention is for adhering and holding the microneedle, and refers to the one prepared by applying a stronger adhesive to a support, as shown in Fig. 20. With this adhesive tape, the microneedle can be adhered and fixed to the skin. The material of the adhesive tape is not particularly limited as long as it is widely used, and commercially available adhesive tapes may be used.

The weaker adhesive of the present invention is for adhering and fixing the support of the adhesive tape described above to the flat plate of the applicator device. Since the weaker adhesive is weaker in adhesion than the adhesive tape, when the adhesive tape is adhered to the skin, the adhesive tape that has been fixed to the flat plate is detached therefrom. As such a weaker adhesive tape, any of commercially available adhesive tapes may be selected appropriately and used, or commercially available double-sided tapes etc. may be used.

The adhesive tape is, together with the pin-frog-shaped microneedle, firmly attached to the elevated skin and detached from the applicator. In this manner, by simply pressing the applicator device of the present invention against the skin, without any other special operations, the pin-frog-shaped microneedle can be adhered to the skin.

The tape-like chip of the present invention has a support of 1 to 50 cm2 and adhesives applied to both faces of the support, which are an adhesive for adhering to the applicator and an adhesive for adhering the microneedle to the skin, and the microneedle is placed on a central portion of the support. Here, the tape-like chip of the present invention is, when the applicator (device) is removed from the skin, required to be detached from the applicator and remain on the skin. The adhesives on both faces of the support each serve to fix the microneedle to the skin, and to store and fix the support to which the microneedle is adhered in the applicator. In addition, the adhesive for storing and fixing the support into the applicator may be applied to the applicator side, instead of being applied to the support.

In order for the tape-like chip of the present invention to be detached from the applicator and remain on the skin, it is necessary that the adhesion of the adhesive at the applicator side is 85 to 90% or less of that of the adhesive at the skin side. When the adhesives used in both faces of the tape-like chip are made of the same material, or their adhesive strengths (N/cm$^2$) are the same, the adhesion is proportional to the adhering area of the adhesive. Therefore, in order for the tape-like chip to remain on the skin and be detached from the applicator, it is necessary that the area of the adhesive for the applicator is 85 to 90% or less of that of the adhesive for the skin.

As the shape of the tape-like chip of the present invention, a circular, quadrangular, or rectangular shape may be used, and preferable examples thereof include circular and rectangular shapes.

The "support" of the present invention may be pulp, glass fibers, a fabric of cotton or the like, or a plastic film made of polyurethane, polyester, nylon, polyethylene, polypropylene, polyvinyl chloride, an ethylene-ethyl acrylate copolymer, polyethylene terephthalate, etc., and may be selected from these appropriately, as needed.

The adhesive of the present invention refers to any of well-known adhesives, and examples thereof include acrylic adhesives made of an acrylic polymer; styrene block copolymers such as a styrene-isoprene-styrene block copolymer and a styrene-butadiene-styrene block copolymer; rubber adhesives such as polyisoprene, polyisobutylene, and polybutadiene; silicon adhesives such as silicon rubber, a dimethylsiloxane-based adhesive, and a diphenylsiloxane-based adhesive; vinyl ether adhesives such as polyvinyl methyl ether, polyvinyl ethyl ether, and polyvinyl isobutyl ether; vinyl ester adhesives such as a vinyl acetate-ethylene copolymer; and polyester adhesives made of a carboxylic component such as dimethyl terephthalate, dimethyl isophthalate, and dimethyl phthalate and a polyhydric alcohol component such as ethylene glycol. As for these adhesives, one kind of them may be used or two or more kinds of them may be blended and used.

The adhesive strength (N/cm$^2$) of the adhesive differs depending on the subject to which the adhesive is attached. For example, according to the results measured by the methods of Figs. 27 and 28, the adhesive strength to a polypropylene flat plate having a smooth flat surface is approximately 21 N/cm$^2$, and the adhesive strength to the skin having an uneven and moist surface is about 1 N/cm$^2$. Therefore, the polypropylene (PP) flat plate is much stronger in adhesive strength of the adhesive than the skin. However, as shown in Fig. 30, when the applicator is pulled diagonally instead of perpendicularly, the adhesive strength (N/cm$^2$) of the adhesive is about one-twentieth compared with the case where the applicator is pulled perpendicularly. For example, the adhesive strength to the polypropylene flat plate is a value of approximately 21 N/cm$^2$ when the applicator is pulled vertically; however, it is decreased to a value of about 1 N/cm$^2$ when the applicator is pulled diagonally.

In order for the support to which the microneedle is placed to be detached from the applicator and remain on the skin, it is necessary to weaken the adhesion between the applicator and the support and strengthen the adhesion between the support and the skin. Thus, according to the method shown in Fig. 31, the adjustment of the strength level of the adhesion was assessed. It was found out that, as for the strength level of the adhesion, when different adhesives are used and their adhesions differ about 10 to 15% or more from each other, the support remains at the stronger adhesive side. Similarly, it was found out that, when the same adhesive is used and the adhered areas of the adhesive differ about 10 to 15% or more from each other, the support remains at the larger adhered area side. Besides, it was found out that the adjustment can be made by applying the adhesive in a shape which allows for easy detachment.

The shape which allows for easy detachment refers to a shape which lessens a load on the portion to which the first load is applied when the support is detached from the applicator. Examples thereof include a shape having an acute angle shape, etc. that triggers the detachment, as shown in Fig. 34. Further, the adjustment can be made for the position where the adhesive is applied. For example, as shown in Fig. 35, even with the same application area of an adhesive, the ease of detachment can be adjusted by changing its application position. In comparison between the case where the adhesive is applied to the inner position from the periphery of the tape-like chip as shown in Fig. 40 (right) and the case where the adhesive is applied to the protruded position from the periphery of the tape-like chip as shown in Fig. 40 (left), it was found out that the case shown in Fig. 40 (right) achieves easier detachment. From this result, examples of preferable application shape and position of the adhesive are those shown in Figs. 36 to 39 and 41. Here, as a preferable position of the adhesive placed, it is desirable that the adhesive is placed at a position away from the periphery of the tape-like chip at a distance of 0.1 mm or more.

In addition, as the adhesive used for this, a double-sided tape may be appropriately cut and used in a desired shape. For example, as shown in Fig. 32, triangular double-sided tapes may be provided at four places to bond the support. Thus, there is provided a tool (clog-shaped applicator), in which a microneedle is fixed to the adhesive tape for the skin, and a support of the adhesive tape is fixed to a flat plate of the applicator by a weaker adhesive member such as a double-sided tape.

As a method of manufacturing the applicator device of the present invention, in the case of the clog-shaped applicator device, rectangular columns are, as the skin fixing members, adhered to both sides of a flat plate member of the applicator. The adhesive used is selected according to the material of the flat plate and the rectangular columns. As a result of this, an applicator, for example, as shown in Figs. 7 and 8, is prepared. Next, a weaker adhesive or a double-sided tape is placed on the flat plate, and an adhesive tape is adhered thereon at a central portion of the applicator. Then, a pin-frog-shaped microneedle is placed thereon at the central portion of the applicator, and thereby an applicator device of the present invention is prepared. As required, the weaker adhesive is applied to the surfaces of the skin fixing members, and a protective sheet is adhered thereto, so as to protect tip portions of small needles of the pin-frog-shaped microneedle.

In addition, the sequence of the manufacturing processes of the applicator device may be changed such that a pin-frog-shaped microneedle is placed on a flat plate member first and thereafter rectangular columns are adhered to both sides of the flat plate member.

When the applicator device of the present invention is pressed against the skin, as shown in Fig. 13, while the skin is pressed down with the skin fixing members, it is elevated in the area defined between the skin fixing members (area defined by the distance a and the distance b). The height of elevation (the distance between the skin fixed by the skin fixing members and the peak of the elevated skin) and the rising direction of the skin (whether or not the skin rises perpendicularly to the microneedle) are measured by the following method and assessed.

As shown in Fig. 16(1), a parallelism measuring jig is manufactured by placing a circular column of 3.5 mm perpendicularly on the center of a circular disk of 15 mm in diameter made of a polypropylene resin. The jig is placed on the skin of the arm. Next, for example, as shown in Fig. 16(2), a clog-shaped applicator having an opening of 8 mm in diameter at a central portion thereof, through which the circular column of the jig can be passed, or, as shown in Fig. 19, a *wappen*-shaped applicator is manufactured. As shown in Fig. 17(1), the circular column of the jig is passed through the opening of the applicator, and the applicator is placed on the skin of the arm. Then, the applicator is pressed down to a certain distance (12 mm) by using a rheometer manufactured by Shimadzu Corporation. In order to press down the applicator, a circular column-shaped rod of 5 mm in diameter is used. Various positions on the flat plate of the applicator (from a central portion to a peripheral portion of the applicator) were pressed down with the rod, and it was measured how the angle between the parallelism measuring jig and the flat plate of the applicator was changed depending on the position where the rod is brought into contact with the flat plate, as shown in Fig. 18. In addition, it was measured how much the skin rose when the applicator was pressed down to a certain distance (12 mm) as described above, with a caliper by using the opening of 8 mm$\phi$.

Regardless of the position of the rod for pressing down (the position on the flat plate where force is applied), the circular column of the jig always keeps a perpendicular relationship to the flat plate. This shows that, as shown in Fig. 17(3), the skin moves coordinately with the applicator. This also shows that, regardless of the position on the flat plate of the applicator where force is applied, the skin surface at the central portion of the applicator rises perpendicularly. Also, the height of rise of the skin (distance) was measured with a caliper. As a result, it was shown that, regardless of the shape of the flat plate of the applicator of the present invention being a square or a rectangle, the applicator does not exhibit so much difference in the height of elevation of the skin as shown in Figs. 3 to 6. It was found out that, although the height of elevation of the skin is not so much affected by the shape of the flat plate as described above, it is considerably affected by the kinds (shapes) of the skin fixing members. That is, the skin is likely to be elevated in the order of the clog-shaped applicator, the four-corner applicator, and the three-point applicator.

In the clog-shaped applicator, it is assumed that the height of elevation of the skin is changed depending on the way to place the applicator; however, in practice, it exhibits no difference even if the way to place the applicator is changed (perpendicular placement and parallel placement with respect to the direction of the arm), as shown in Tables 1 and 2.

Here, the clog-shaped applicator of the present invention is used, and the device on which the microneedle is placed as shown in Fig. 32 is used. Then, for example, as shown in Fig. 33, by pressing the pin-frog-shaped applicator and thereafter removing the device, the microneedle can remain on the skin and be fixed to the skin easily.

In addition, a drug is applied to the pin-frog-shaped applicator used in the present invention. The drug to be applied is not particularly limited as long as it has been used for the treatment. When the drug is a biopolymer such as a protein, an antigen, an antibody, etc., it can be applied to the microneedle as an aqueous solution since its solubility in water is high. When the drug is a low molecular weight compound such as an antibiotic or an antipsychotic drug, it may be applied to the microneedle as an aqueous solution depending on its solubility, or may be applied thereto by the use of an organic solvent solution.

When a certain amount of drug is necessary to exhibit the effect, it is possible to allow small needles of the microneedle to support the necessary amount of drug by repeating the steps of immersing the small needles in an aqueous solution of the drug etc. and drying them.

**EXAMPLES**

The present invention will be explained in detail with reference to Examples; however, it is not limited to any of the following Examples.

(Example 1) Manufacture of Applicators

(1) Manufacture of Clog-shaped applicator

To a peripheral portion of a rectangular flat plate (30 × 40 mm, 2 mm in thickness) made of acrylic were adhered side faces (5 × 30 mm) of two rectangular columns (3 × 5 × 30 mm) made of acrylic as shown in Fig. 7, to form skin fixing members. Thus, a clog-shaped applicator of 3 mm in height was prepared. A perspective view of the obtained clog-shaped applicator is shown in Fig. 8. The area surrounded by the two rectangular columns (skin fixing members) (a portion in which the skin was to be elevated) was 30 × 30 mm.

(2) Manufacture of Four-corner Applicator (a)

To four corners of a square flat plate (30 × 30 mm, 2 mm in thickness) made of acrylic were adhered top portions of four hemispheres (3 mm in diameter) made of acrylic as shown in Fig. 9, to form skin fixing members. Thus, a four-corner applicator having hemispheres of 3 mm in height at four corners was prepared. A perspective view of the obtained four-corner applicator is shown in Fig. 10. The area surrounded by the four hemispheres (a portion in which the skin was to be elevated) was 24 × 24 mm.

(3) Manufacture of Four-corner Applicator (b)

To four corners of a square flat plate (20 × 20 mm, 2 mm in thickness) made of polypropylene were fusion-bonded top portions of four rectangular columns (5 × 5 × 10 mm) made of polypropylene as shown in Fig. 1(2), to form skin fixing members. Thus, a four-corner applicator having rectangular columns of 10 mm in height at four corners was prepared. A perspective view of the obtained four-corner applicator is shown in Fig. 11. The area surrounded by the four rectangular columns (a portion in which the skin was to be elevated) was 10 × 10 mm.

(4) Manufacture of Three-point Applicator

To the center of a peripheral portion of a square flat plate (20 × 20 mm, 2 mm in thickness) made of polypropylene was fusion-bonded a top portion of one rectangular column (5 × 5 × 10 mm) made of polypropylene as shown in Fig. 1(3), and to both ends 10 mm away from this rectangular column were adhered rectangular columns (5 × 5 × 10 mm) as shown in Fig. 1(3), to form skin fixing members. Thus, a three-point applicator having rectangular columns of 10 mm in height at three places was prepared. A perspective view of the obtained three-point applicator is shown in Fig. 12. The area surrounded by the three rectangular columns (a portion in which the skin was to be elevated) was 1 × 0.9 mm.

(Example 2) Manufacture of Various Applicators and Distance of Elevation on Skin during Pressing

Various applicators shown in Fig. 1 were prepared according to Example 1. These applicators were used and placed on the skin of the arm and then pressed down about 12 mm from the placed position so that they were pressed against the skin. Then, the height of elevation of the skin (the distance of elevation of the skin) was measured with a caliper. As shown in the following Tables 1 to 5, there were prepared various devices in which the kinds of applicators and the size of the skin area surrounded by skin fixing members were changed. Here, the height of the skin fixing members was selected appropriately according to the size of the area surrounded by the skin fixing members.

(1) Clog-shaped Applicator

Various types of clog-shaped applicators prepared according to Example 1(1) were used and pressed against the skin. Then, the height of elevation of the skin (the distance of elevation of the skin) was assessed. Here, when the applicator was pressed against the skin, there were two cases where the fixing members were placed perpendicularly to the direction of the arm and pressed (perpendicular placement) as shown in Figs. 2 (left) and 13 and where they were placed parallel to the direction of the arm and pressed (parallel placement) as shown in Fig. 2 (right). The difference of the perpendicular placement and the parallel placement brought about the difference in the initial height of the skin surface when the applicator was placed on the skin. Thus, the actual height (distance) of elevation of the skin when the applicator was pressed against the skin was measured. The result is classified and shown in the following Table 1 (perpendicular placement) and Table 2 (parallel placement), as well as Fig. 3 (perpendicular placement) and Fig. 4 (parallel placement).

[Table 1]

| Shape flat plate | of Side a (mm) | Side b (mm) | Height of elevation (mm) |
|---|---|---|---|
| Square | 5 | 5 | 1 |
| | 10 | 10 | 2 |
| | 15 | 15 | 2.5 |
| | 20 | 20 | 3.5 |
| | 25 | 25 | 5 |
| | 30 | 30 | 6 |
| | 35 | 35 | 6 |
| | 70 | 70 | 11 |
| Rectangle | 10 | 20 | 3.5 |
| | 10 | 30 | 6 |
| | 20 | 10 | 2 |
| | 30 | 10 | 2 |
| | 40 | 70 | 11 |

[Table 21

| Shape of flat plate | Side a (mm) | Side b (mm) | Height of elevation (mm) |
|---|---|---|---|
| Square | 5 | 5 | 1 |
| | 10 | 10 | 2 |
| | 15 | 15 | 2.5 |
| | 20 | 20 | 3.5 |
| | 25 | 25 | 5 |
| | 30 | 30 | 6 |
| | 35 | 35 | 6 |
| Rectangle | 10 | 20 | 3.5 |
| | 10 | 30 | 6 |
| | 20 | 10 | 2 |
| | 30 | 10 | 2 |

As shown in Tables 1 and 2, regardless of the way to place the applicator (perpendicular placement, parallel placement), there was no difference in the actual height of elevation.

(2) Four-corner Applicator

Various types of four-corner applicators prepared according to Example 1(3) were used and pressed against the skin. Then, the height of elevation of the skin (the distance of elevation of the skin) was assessed. When a square four-corner applicator was used, the height of elevation of the skin (the distance of elevation of the skin) was as in Table 3.

On the other hand, when a rectangular four-corner applicator was used, there were two cases where the longer sides were placed perpendicularly to the direction of the arm and pressed (perpendicular placement) and where they were placed parallel to the direction of the arm and pressed (parallel placement) as shown in Fig. 2 (left) and (right). The result obtained by distinguishing between the perpendicular placement and the parallel placement of the rectangular four-corner applicator is summarized in Table 4, as well as in Fig. 5.

[Table 3]

| Shape of flat plate | Side a (mm) | Side b (mm) | Height of elevation (mm) |
|---|---|---|---|
| Square | 5 | 5 | 2.5 |
| | 10 | 10 | 3.5 |
| | 15 | 15 | 6.5 |
| | 20 | 20 | 9 |
| | 30 | 30 | 11 |
| | 40 | 40 | 15 |

[Table 4]

| Shape of flat plate | Side a (mm) | Side b (mm) | Height of elevation (mm) |
|---|---|---|---|
| Rectangle | | | |
| (perpendicular placement) | 10 | 15 | 7.5 |
| | 20 | 15 | 7.5 |
| (parallel placement) | 10 | 15 | 5 |
| | 20 | 15 | 7.5 |

The result of Fig. 5 obtained by the result of Tables 3 and 4 showed that, regardless of change in the size and shape of the flat plate of the device or the way to place the device, there was a positive relationship between the distance of the side b and the height of elevation of the skin (distance of elevation).
Here, the height of elevation was found to be higher (longer distance) than that of the clog-shaped applicator.
Further, since the skin fixing members are four rectangular columns, corners of the rectangular columns were brought into contact with the skin when pressed against the skin, thereby causing pain. Therefore, in order to alleviate the pain, the corners of the rectangular columns were rounded.

(3) Three-point Applicator

The three-point applicator prepared in Example 1(4) was used and pressed against the skin. Then, the height of elevation of the skin (the distance of elevation of the skin) was assessed. The result is shown in Table 5 and Fig. 6.
[Table 5]

| Shape of flat plate | Side a (mm) | Side b (mm) | Height of elevation (mm) |
|---|---|---|---|
| Square | 5 | 5 | 2 |
| | 10 | 10 | 3 |
| | 20 | 20 | 7.5 |
| | 30 | 30 | 10 |
| | 30 | 30 | 11 |
| | 40 | 40 | 15 |

As shown in the result of Table 5, the height of elevation of the skin was the longest in the three-point applicator. In this manner, it was shown that the smaller the skin area pressed by the skin fixing members was, the longer the height of elevation of the skin was. Also, it was shown that the four-corner applicator and the three-point applicator were not so affected by the placement direction with respect to the arm (perpendicular placement, parallel placement), compared with the clog-shaped applicator.

(Example 3) Preparation of Various Microneedle Devices (1) Clog-shaped Microneedle Device

To the clog-shaped applicator of Fig. 8 prepared in Example 1(1), as shown in Fig. 20, was placed a circular tape with four triangular double-sided tapes. Then, at the center of the circular tape of 23 mm was placed a circular pin-frog-shaped microneedle of 10 mm in diameter. Thus, a clog-shaped microneedle device shown in Fig. 14 was obtained.

(2) Four-corner Microneedle Device

To the clog-shaped applicator of Fig. 10 prepared in Example 1(2), in the same manner as in the above (1), was placed a circular tape with four triangular double-sided tapes. Then, at the center of the circular tape of 23 mm was placed a circular pin-frog-shaped microneedle of 10 mm in diameter. Thus, a four-corner microneedle device shown in Fig. 15 was obtained.

(Example 4) Preparation of Microneedle Device Having Protrusion for Pressing

The result of Test Example 1 showed that it was not preferred to press a portion of a top plate of the applicator close to a position where the skin fixing member was not present, from the viewpoint of the puncture properties. Therefore, in order to press the central portion of the top plate or apply uniform force to the skin fixing members, a protrusion (structure) for pressing such as the ones shown in Figs. 23 to 26 was placed on the top plate of the applicator.

(1) Microneedle Device with Protrusion for Pressing Placed on Central Portion of Top Plate of Clog-shaped Applicator

As shown in Figs. 23 and 24, an applicator was prepared with a circular column-shaped or hemispherical-shaped protrusion placed on a central portion of a top plate of a clog-shaped applicator. As a result of this, easier pressing and stable puncture properties were obtained.

(2) Microneedle Device with Arched Structure Placed on Top Plate, for Applying Uniform Force to Skin Fixing Members of Clog-shaped Applicator

A microneedle device as shown in Fig. 25 can be obtained by placing an arched structure on a top plate of a clog-shaped applicator. As the arched structure, for the reinforcement, an auxiliary plate can be added to a central portion of the arch. As a device having such a shape, a microneedle device with an arched structure placed on a top plate of a clog-shaped applicator as shown in Fig. 26 was prepared. As a result of this, easier pressing and stable puncture properties were obtained.

(Example 5) Manufacture of Applicator that Stores and Fixes Tape-like Chip on which Microneedle is Placed

(1) Manufacture of Tape-like Chip, to Both Faces of which Adhesives are Plastered

As a tape-like chip, CATHEREEP FS Roll (φ 23) manufactured by Nichiban CO., LTD. was used. A paper double-sided tape (NICETACK NW-40) was cut into four isosceles right triangles of 5 × 5 mm, and these four pieces were placed on a top portion of the CATHEREEP FS Roll, as shown in Fig. 30.
Thus, a tape-like chip, to both faces of which adhesives were adhered, was manufactured.

(2) Manufacture of Applicator that Stores and Fixes Tape-like Chip

As an applicator, an applicator made of acrylic as shown in Figs. 7 and 8 was used. The tape-like chip according to the above (1) was placed on a central portion of the applicator and fixed to the applicator with the paper double-sided tape described above.
Thus, an applicator on which the tape-like chip was placed was manufactured.

(3) Manufacture of Applicator Fixing Tape-like Chip on which Microneedle is Placed

Protector paper on the adhesive of the CATHEREEP FS Roll in the applicator obtained in (2) was peeled off, and a microneedle (φ 10 mm) was placed on a central portion thereof.
Thus, as shown in Fig. 32, an applicator fixing the tape-like chip on which the microneedle was placed was manufactured.
The manufactured applicator was placed on the skin of the upper arm, and pressed in the direction of the arrow as shown in Fig. 33. With the pressing, the skin was elevated and the microneedle was inserted into the skin perpendicularly, so that the adhesive of the CATHEREEP FS Roll was adhered to the skin. Thereafter, the applicator was pulled up in the direction of the arrow. Thus, this made it possible to remove only the applicator therefrom and retain the tape-like chip, on which the microneedle was placed, on the skin.

(Example 6) Manufacture of Tape-like Chip with Adhesive Applied to Both Faces of Support in Various Shapes

According to the result of Test Example 6, when a double-sided tape (adhesive A) for adhering an applicator and a tape-like chip has an area about 90% or less of that of an adhesive adhering to the skin, the applicator and the tape-like chip are detached from each other at the portion of the adhesive A. Further, the result of Test Example 7 showed that the shape, number, and location of the adhesive A contributes to the ease of detachment. Therefore, the area of the adhesive A was set to be about 90% or less of that of the adhesive adhering to the skin, and the shape of the adhesive A was changed to prepare various tape-like chips.
As the shape of the adhesive A, various shapes shown in Figs. 34 to 41 can be employed.

(Test Example 1) Assessment Test of Stable Puncture Efficiency on Applicator Device

The various microneedle devices prepared in Example 3 were used, and it was confirmed whether or not the microneedle entered the skin perpendicularly. Further, it was studied which position of the flat plate of the device was required to receive the pressing force, in order for the microneedle to enter the skin perpendicularly.

(1) Angle of Microneedle during its Skin Puncture, Resulting When Pressing is Made in Position Shifted from Center of Device in Direction (Perpendicular) of Skin Fixing Members

For this object, as shown in Fig. 16, a clog-shaped applicator (the area surrounded by the skin fixing members was 30 × 30 mm, 7 mm in height) according to Example 1(2) was prepared. Then, an opening of 8 mm in diameter was provided at the center of the flat plate. A circular column of 3.5 mm in diameter was placed upright on the center of a circular disk of 15 mm in diameter, and this was placed on the skin as shown in Fig. 17. Then, the circular column was passed through the hole of the clog-shaped applicator and overlapped together, and the top of the flat plate of the applicator was pressed with a rod of 5 mm in diameter. The change of angle between the flat plate of the applicator and the circular column placed on the skin, which occurred during the pressing of the applicator, was assessed as shown in Fig. 18.
Specifically, the position on the flat plate pressed with the rod of 5 mm in diameter and the pressed distance of the rod were changed. Then, by assessing the change of angle, it was assessed how the skin surface was elevated.
The result is shown in Table 6.
[Table 6]

| Pressing distance of rod (mm) | Pressed position (shift from the center) | | | | | |
| | Place fixing members in parallel direction | | | Place fixing members in perpendicular direction | | |
| | 6 mm | 9 mm | 12 mm | 6 mm | 9 mm | 12 mm |
| 0 | 90 degree | 90 degree | 90 degree | 90 degree | 90 degree | 90 degree |
| 1 | 90 degree | 90 degree | 90 degree | 90 degree | 90 degree | 90 degree |
| 2 | 90 degree | 90 degree | 90 degree | 90 degree | 90 degree | 90 degree |
| 4 | 90 degree | 90 degree | 90 degree | 90 degree | 90 degree | 90 degree |
| 6 | 90 degree | 90 degree | 90 degree | 90 degree | 90 degree | 90 degree |
| 8 | 90 degree | 90 degree | 90 degree | 90 degree | 90 degree | 90 degree |

(continued)

| Pressing distance of rod (mm) | Pressed position (shift from the center) | | | | | |
|---|---|---|---|---|---|---|
| | Place fixing members in parallel direction | | | Place fixing members in perpendicular direction | | |
| | 6 mm | 9 mm | 12 mm | 6 mm | 9 mm | 12 mm |
| 10 | 90 degree | 90 degree | 90 degree | 90 degree | 90 degree | 90 degree |
| 12 | 90 degree | 90 degree | 90 degree | 90 degree | 90 degree | 90 degree |
| [Note]<br>Place fixing members in parallel direction: as shown in Fig. 2(b), the fixing members were placed in a parallel direction with respect to the arm (applicator parallel placement).<br>Place fixing members in perpendicular direction: as shown in Fig. 2(a), the fixing members were placed in a perpendicular direction with respect to the arm (applicator perpendicular placement).<br>Pressed position (shift from the center): the position to be pressed was set at a position where a distance of 6, 9, or 12 mm was away from the center of the applicator in a direction (perpendicular) of the fixing members. | | | | | | |

According to the result shown above, when a position out of the center of the applicator (a position shifted from the center of the device in a direction (perpendicular) of the skin fixing members) was pressed, it seemed that the applicator was tilted. However, it was found out that, in coordination with the tilt, the skin surface was also tilted, and the flat plate of the applicator and the skin were always kept parallel to each other.
That is, regarding the pin-frog-shaped microneedle, it was shown that the skin was elevated during the pressing while keeping the perpendicular position.

(2) Angle of Microneedle during its Skin Puncture Resulting When Pressing is Made in Position Shifted from Center of Device in Parallel Direction of Skin Fixing Members

For this object, as shown in Fig. 22, a clog-shaped applicator (the area surrounded by the skin fixing members was 30 × 30 mm, 7 mm in height) according to Example 1(2) was prepared. As shown in Fig. 22, a rectangular hole (cavity) of 7 × 18 mm was provided at the position right side from the center of the flat plate. A circular column of 3.5 mm in diameter was placed upright on the center of a circular disk of 15 mm in diameter, and this was used as a parallelism measuring jig and placed on the skin. Then, the circular column was passed through the hole of the clog-shaped applicator and overlapped together, and the top of the flat plate of the applicator was pressed with a rod of 5 mm in diameter. The change of angle between the flat plate of the applicator and the circular column placed on the skin, which occurred during the pressing of the applicator, was assessed as shown in Fig. 18.
In the same manner as in the above, the position on the flat plate pressed with the rod and the pressing distance of the rod were changed. Then, by assessing the change of angle shown by the parallelism measuring jig, it was assessed how the skin surface was elevated.
The result is shown in Table 7.
[Table 7]

| Pressing distance of rod (mm) | Pressed position (shift from the center) (mm) | | |
|---|---|---|---|
| | 3 | 6 | 9 |
| 0 | 90 degree | 90 degree | 87 degree |
| 1 | 90 degree | 90 degree | 85 degree |
| 2 | 90 degree | 90 degree | 80 degree |
| 4 | 90 degree | 90 degree | |
| 6 | 90 degree | 90 degree | |
| 8 | 90 degree | 85 degree | |
| 10 | 90 degree | | |
| 12 | 90 degree | | |

As shown in Table 7, when pressing was carried out at the position 3 mm shifted from the center of the device in a parallel direction of the skin fixing members, the flat plate of the applicator and the skin were kept parallel to each other; however, when the pressing was carried out at the position 6 mm shifted therefrom, they could not be kept parallel to each other.

This result is significantly different from that of Table 6, and in the case of the clog-shaped applicator, it is necessary to consider the position to be pressed. Thus, it is considered that in the case of the clog-shaped applicator, a protrusion (structure) for pressing should be placed on the flat plate (central portion) of the applicator.

On the other hand, as shown in Test Example 2, in the case of a *wappen*-shaped applicator, the skin with which the applicator is brought into contact is surrounded by, for example, a circular shape; therefore, regardless of the position of the applicator to be pressed, the flat plate of the applicator and the skin are kept parallel to each other. That is, it is considered that, in the case of a clog-shaped applicator, since the skin is fixed with two parallel plates, when the pressing is carried out at a position where there is no member for fixing the skin, it is difficult to keep the flat plate of the applicator and the skin parallel to each other (it is difficult to allow the microneedle to enter the skin perpendicularly), as described above.

(Text Example 2) Assessment Test of Puncture Efficiency on Other Applicator Device

In the same manner as in Test Example 1, a *wappen*-shaped applicator as shown in Fig. 19 was prepared. Then, the parallelism between the skin surface and the flat plate of the applicator was assessed. A hole of 8 mm in diameter was provided at the center of the *wappen*-shaped applicator. In the same manner as in Test Example 1, the pressing was carried out at a position away from the center with a rod of 5 mm in diameter, and then the change of angle between the circular column provided on the skin surface and the flat plate of the applicator was assessed. That is, the position on the flat plate pressed with the rod and the pressing distance of the rod were changed. Then, by assessing the change of angle, it was judged how the skin surface was elevated. The result is shown in Table 8.

[Table 8]

| Pressing distance of rod (mm) | Pressed position (shift from the center) | | |
|---|---|---|---|
| | Perpendicular direction to a side of square | | |
| | 5 mm | 7.5 mm | 9 mm |
| 0 | 90 degree | 90 degree | 90 degree |
| 1 | 90 degree | 90 degree | 90 degree |
| 2 | 90 degree | 90 degree | 90 degree |
| 4 | 90 degree | 90 degree | 90 degree |
| 6 | 90 degree | 90 degree | 90 degree |
| 8 | 90 degree | 90 degree | 90 degree |
| 10 | 90 degree | 90 degree | 90 degree |
| 12 | 90 degree | 90 degree | 90 degree |

In the same manner as in Test Example 1, it was shown that, in the case of the applicator having a circular surrounding area, the skin was elevated during the pressing, while keeping the perpendicular position. Thus, it was shown that the use of the *wappen*-shaped applicator allowed the microneedle to enter the skin perpendicularly.

(Text Example 3) Assessment Test of Puncture Property on Applicator Device

The clog-shaped applicator device prepared in Example 3(1) was used to carry out a puncturing test to the skin. As an assessment method, a human skin model of Fig. 21 was employed. First, on a rubbery flat plate made of a styrene-isoprene-styrene block copolymer (SIS), a rat skin (5 weeks male, abdominal part) was placed, and thereon the microneedle device prepared in Example 1 was placed. The device was pressed 12 mm with a rheometer manufactured by Shimadzu Corporation.

After the device and the microneedle were removed therefrom, the skin surface of the rat was stained with a gentian violet solution and observed. The punctured portions of the skin were stained with blue, making the punctured portions visible.

According to the result of the puncture, on the rat skin, portions punctured with small needles were shown as blue spots in a regular form.

In addition, changes such as breakage of small needles of the microneedle after the puncture were assessed. There was no considerable change in the shape of the small needles, and no breakage, bending sideways, etc were observed. Thus, the applicator device of the present invention can enter the skin appropriately.

(Test Example 4) Assessment Test of Tensile Stress on Adhesive to Human Skin and Polypropylene Flat Plate

(1) Tensile Stress of Adhesive to Polypropylene Flat Plate

As for the adhesion (tensile stress) of an adhesive, the maximum stress of the adhesion was measured with a stress measuring device (Rheometer EZ-Test manufactured by Shimadzu Corporation).

Polypropylene flat plates of $3 \times 3$ cm $\times$ 2.5 mm were laminated, and a PET sheet of 3 cm wide, 25 cm long, and 0.1 mm thick was interposed and adhered therebetween, and thus integrated. As shown in Fig. 27, the PET sheet was fixed to a tension test jig placed on the rheometer. Just below the polypropylene flat plate (A) was horizontally placed a polypropylene flat plate (B) having the same size as (A). These two upper and lower polypropylene flat plates were adhered by means of an adhesive (NICETACK NW-40, manufactured by Nichiban CO., LTD.). The lower one of the adhered flat plates, namely (B), was fixed, and as shown in Fig. 27, the tension jig was elevated to pull up the upper polypropylene flat plate (A) vertically. Then, the maximum stress when the upper and lower polypropylene flat plates were detached from each other was measured.

Here, the size of the adhesive (20, 15, 10, 5 mm$\phi$, $14 \times 14$ mm, and $0.3 \times 0.3$ mm) was changed, and the maximum stress of each case at the time of detachment was measured.

The result is summarized in Table 9.

[Table 9]

| No. | Adhesive (diameter or sides) | Area (cm$^2$) | Maximum stress (N) | N/cm$^2$ |
|-----|------------------------------|---------------|---------------------|----------|
| 1 | 20 mm$\phi$ | 3.14 | 45 | 14.3 |
| 2 | 14 x 20 mm | 2.80 | 40 | 14.3 |
| 3 | 15 mm$\phi$ | 1.77 | 31 | 17.5 |
| 4 | 10 mm$\phi$ | 0.79 | 15 | 19.0 |
| 5 | 5 mm$\phi$ | 0.20 | 5.2 | 26.0 |
| 6 | 0.3 x 0.3 mm | 0.09 | 3.2 | 35.6 |

As shown in Table 9, the larger the adhered area is, the smaller the stress per unit is, with a tendency to converge to 14.3 N/cm$^2$.

(2) Tensile stress of Adhesive to Human Skin

In the same manner as in the above (1), the adhesion of the adhesive (tensile stress) was measured. Here, the human upper arm was used instead of the polypropylene flat plate (B), and the assessment test of the tensile stress was carried out as shown in Fig. 28.

The result is summarized in Table 10.

[Table 10]

| No. | Adhesive (diameter or sides) | Area (cm$^2$) | Maximum stress (N) | N/cm$^2$ |
|-----|------------------------------|---------------|---------------------|----------|
| 1 | 20 mm$\phi$ | 3.14 | 3.7 | 1.18 |
| 2 | 14 x 20 mm | 2.80 | | |
| 3 | 15 mm$\phi$ | 1.77 | 1.8 | 1.02 |
| 4 | 10 mm$\phi$ | 0.79 | 0.9 | 1.14 |

(continued)

| No. | Adhesive (diameter or sides) | Area (cm$^2$) | Maximum stress (N) | N/cm$^2$ |
|---|---|---|---|---|
| 5 | 5 mm$\phi$ | 0.20 | | |
| 6 | 0.3 x 0.3 mm | 0.09 | | |

Comparison between the results (stress/cm$^2$) of Table 9 and Table 10 shows that the adhesive is likely to be detached from the human skin compared with the polypropylene resin. As a reason, it is considered that, the polypropylene flat plate is smooth and thus the adhesive is likely to be adhered thereto uniformly and less likely to be detached therefrom; however, the skin is finely uneven and has moisture, sebum, etc. and thus the adhesive is less likely to be adhered thereto uniformly.
Thus, comparison is made between the adhesion (stress/cm$^2$) of the adhesive to the human skin and the adhesion (stress/cm$^2$) of the adhesive to the polypropylene (PP) flat plate, and shown in the following Table 11.
[Table 11]

| No. | (diameter or sides) | Adhesion to human skin (N/cm$^2$) | Adhesion to flat plate of pp (N/cm$^2$) | Adhesion to human skin / Adhesion to flat plate of PP (%) |
|---|---|---|---|---|
| 1 | 20 mm$\phi$ | 1.18 | 14.3 | 8.3 |
| 2 | 14 x 20 mm | | 14.3 | |
| 3 | 15 mm$\phi$ | 1.02 | 17.5 | 5.8 |
| 4 | 10 mm$\phi$ | 1.14 | 19.0 | 6.0 |
| 5 | 5 mm$\phi$ | | 26.0 | |
| 6 | 0.3 x 0.3 mm | | 35.6 | |
| | | | | (Average) 6.7 |

As shown in the result of Table 11, when the adhesion (stress/cm$^2$) of the adhesive to the PP flat plate is regarded as 1, the adhesion (stress/cm$^2$) of the adhesive to the human skin is in a range of approximately 6%. The result of Table 11 is also shown in Fig. 29.

(Test Example 5) Assessment Test of Vertical Tensile Stress When Adhesives Applied to Both Faces of Tape-like Chip are Different from Each Other

When adhesives applied to both faces of a tape-like chip are different in the strength level of the adhesion, detachment occurred at the side with weaker adhesion. Thus, it was studied and assessed how much difference in the strength level of the adhesion was required in order for the detachment to occur at the side with weaker adhesion.

(1) Assessment of Adhesion Difference and Detached Portion on Both Faces of Polypropylene (PP) Flat Plate

As a model experiment, as shown in Fig. 27, an adhesive A and an adhesive B were applied to both faces of a chip of a PP flat plate (3 x 3 cm x 5 mm). In the same manner as in Test Example 4, the chip was interposed and adhered between PP flat plates A and B, and the PP flat plate A was fixed to a tension jig. The jig was raised, and it was confirmed whether detachment occurred at the portion of the adhesive A or the portion of the adhesive B.
As for the adhesive A, NICETACK NW-40 manufactured by Nichiban CO., LTD. was used, and the adhered area of the adhesive was changed as shown in Table 11. As for the adhesive B, while the same NICETACK NW-40 manufactured by Nichiban CO., LTD. was used, the adhered area of the adhesive was fixed at 20 mm$\phi$.
The result is shown in the following Table 12.
[Table 12]

| No. | Adhesive A (diameter or sides) | Adhesive B (diameter or sides) | Area ratio of adhesive A to B (A/B) | Detached portion: Adhesive A or B |
|---|---|---|---|---|
| 1 | 20 mmφ | 20 mmφ | 100 % | B |
| 2 | 19.5 mmφ | 20 mmφ | 95.1 % | B |
| 3 | 14 x 20 mm | 20 mmφ | 89.2 % | A |
| 4 | 15 mmφ | 20 mmφ | 56.3 % | A |
| 5 | 10 mmφ | 20 mmφ | 25.0 % | A |
| 6 | 5 mmφ | 20 mmφ | 6.3 % | A |
| 7 | 0.3 x 0.3 mm | 20 mmφ | 2.9 % | A |

When the same adhesive is applied or adhered to the top and bottom of the tape-like chip but the adhering area differs, since the adhesion is represented by "Tensile stress per 1 cm$^2$" $\times$ "Area", the strength level of the adhesion is represented by the area ratio. As shown in Table 12, when there was a difference in adhesion of about 10% between the adhesive A and the adhesive B, it was shown that detachment occurred certainly at the portion of the adhesive A.

(2) Assessment of Adhesion Difference and Detached Portion on Both Faces of PP Flat Plate in the Case of Human Skin

As shown in Fig. 3, the human forearm was used instead of the PP flat plate C. In the same manner as in the above (1), the detachment test was carried out.
The result is shown in the following Table 13.
[Table 13]

| No. | Adhesive A (diameter or sides) | Adhesive B (diameter or sides) | Area ratio of adhesive A to B (A/B) | Detached portion: Adhesive A or B |
|---|---|---|---|---|
| 1 | 20 mmφ | 20 mmφ | 100 % | B |
| 2 | 19.5 mmφ | 20 mmφ | 95.1 % | B |
| 3 | 14 x 20 mm | 20 mmφ | 89.2 % | B |
| 4 | 15 mmφ | 20 mmφ | 56.3 % | B |
| 5 | 10 mmφ | 20 mmφ | 25.0 % | B |
| 6 | 5 mmφ | 20 mmφ | 6.3 % | B |
| 7 | 0.4 x 0.4 mm | 20 mmφ | 5.1 % | B |
| 8 | 0.3 x 0.3 mm | 20 mmφ | 2.9 % | A |

As shown in Table 13, in No. 8, for the first time, detachment occurred at the portion of the adhesive A, and the PP flat plate remained on the skin. Before No. 8, detachment occurred at the portion of the adhesive B (skin side). On the other hand, the result of Table 11 shows that the adhesive strength of the adhesive adhered to the skin is decreased to approximately 6% compared with that of the adhesive adhered to the PP flat plate. That is, when the adhesive of the same material is used, in order to compare with the PP flat plate, about 6% of the area of the adhesive B adhering to the skin is matched.
Thus, the result is converted into comparison between PP flat plates as shown in Fig. 27, and this can be shown in the following Table 14.
[Table 14]

| No. | Adhesive A (diameter or sides) | Adhesive B × about 6% (cm$^2$) | Area ratio of adhesive A to B (A/B) | Detached portion: Adhesive A or B |
|---|---|---|---|---|
| 1 | 20 mmφ | about 0.19 | 100% or more | B |
| 2 | 19.5 mmφ | (same as above) | 100 % or more | B |
| 3 | 14 x 20 mm | (same as above) | 100 % or more | B |
| 4 | 15 mmφ | (same as above) | 100 % or more | B |
| 5 | 10 mmφ | (same as above) | 100 % or more | B |
| 6 | 5 mmφ | (same as above) | 100 % or more | B |
| 7 | 0.4 x 0.4 mm | (same as above) | about 84 % | B |
| 8 | 0.3 x 0.3 mm | (same as above) | about 47 % | A |

The results of Tables 12 and 14 show that, in the case where the adhesive A and the adhesive B are applied to both faces of the chip-like tape and there is a difference in the adhesion strength (or in the case where the same material is used for the adhesive and there is a difference in the adhering area), when the difference of the adhesion exceeds 10 to 15%, detachment occurs tidily in the portion of the adhesive with weaker adhesion.

Therefore, in order to bond the tape-like chip to the skin and detach the tape-like chip from the PP device in which the tape-like chip has been stored, the following is found out from the above result:

```
"Adhesion of adhesive A" < "Adhesion of Adhesive B" × (85
to 90%).
```

Further, when the adhesive of the same material is used, the adhering area of the adhesive can be regarded as the adhesion. Since the result of Table 11 shows that the adhesion to the skin is about 6% of the adhesion between the PP flat plates, the above equation can be rewritten as follows and the following relationship is found out:

```
"Plastered area of adhesive A" < ("Skin attached area of
adhesive B" × about 6%) × (85 to 90%).
```

(Test Example 6) Assessment Test of Diagonal Tensile Stress on Adhesives Applied to Both Faces of Tape-like Chip

In Test Example 5, as shown in Figs. 27 and 28, the PP plate A was pulled up vertically with a stress measuring device (Rheometer EZ-Test manufactured by Shimadzu Corporation), and the detachment test on the adhesives applied to both faces of the PP plate A was carried out. Then, it was studied how much difference in the strength level of the adhesion was required in order for the detachment to occur at the side with weaker adhesion.
Here, in Test Example 6, as shown in Fig. 30, it was assessed how much tensile stress was alleviated by pulling up the PP plate A diagonally.

(1) Preparation of Tape-like Chip with Adhesive Plastered Thereto

As shown in Fig. 30, as a tape-like chip, CATHEREEP FS Roll (φ 23) manufactured by Nichiban CO., LTD. was used. On the top portion thereof (PP plate A side), an adhesive A (NICETACK NW-40) was adhered at four places (four isosceles right triangles of 5 x 5 mm).
The attached area to the skin was about 415 mm2, and the attached area to the PP plate A was 50 mm2.
Therefore, based on the result of Test Example 5 (Vertical pulling up), these numbers were applied to the following equation.

$$\text{``Plastered area of adhesive A''} < (\text{``Skin attached area of}$$
$$\text{adhesive B''} \times \text{about 6\%}) \times (85 \text{ to } 90\%)$$

$$(50 \text{ mm}^2) > (25 \text{ mm}^2) \times (85 \text{ to } 90\%)$$

In the case of vertical pulling up as in Test Example 5, it was considered that detachment occurred at the portion of the skin side.

(2) Assessment of Decreased Tensile Stress by Pulling up Diagonally

As shown in Fig. 30, the chip-like tape of the above (1) was adhered to the skin. Then, a PP plate A was placed and connected to a stress measuring device (Rheometer EZ-Test manufactured by Shimadzu Corporation). By pulling up a jig of the stress measuring device to pull up the PP plate A diagonally, the tensile stress when the adhesive A attached to the PP plate was detached therefrom was assessed. The result is shown in the following Table 15.
[Table 15]

| No. | Area of Adhesive A Triangle shape, 4 pieces) | Tensile stress diagonally (N) | Tensile when pulled up vertically | Decrease tensile stress |
|---|---|---|---|---|
| 1 | 12.5 x 4 mm$^2$ | 0.5 | | |
| 2 | (same as above) | 0.5 | | |
| 3 | (same as above) | 0.5 | | |
| | Average | 0.5 (1 N/$^2$) | about 20 N/cm$^2$ (from result of Table 1) | about 95% decrease |

In the experiment of Table 15, detachment of the adhesive at the skin side did not occur at all. As shown in the result of Table 15, by pulling up the PP plate A diagonally, the tensile stress decreased by about 95% and the stress was about 5%.

Therefore, when pulling up diagonally, in order that detachment of the adhesive B at the skin side does not occur and detachment of the adhesive A occurs, it is necessary to satisfy the following equation.

$$\text{``Adhesion of adhesive A''} \times \text{about 5\%} < \text{``Adhesion of}$$
$$\text{adhesive B''} \times (85 \text{ to } 90\%)$$

Further, when the adhesive of the same material is used, the adhering area of the adhesive can be regarded as the adhesion. Since the result of Table 11 shows that the adhesion to the skin is about 6% of the adhesion between the PP flat plates, the above equation can be rewritten as follows:

$$\text{``Plastered area of adhesive A''} \times \text{about 5\%} < (\text{``Skin}$$
$$\text{attached area of adhesive B''} \times \text{about 6\%}) \times (85 \text{ to } 90\%).$$

Further, this equation is simplified to obtain the following approximate equation.

"Plastered area of adhesive A" < "Skin attached area of adhesive B" × about 90%

(Test Example 7) Verification of Ease of Transfer of Tape-like Chip to Skin depending on Shapes of Adhesive

Applicators with a tag (Fig. 33(c)) to which a tape-like chip was fixed with shapes of the adhesive shown in Fig. 34 were formed. The applicators were used in the human forearm by the method of pulling up diagonally shown in Fig. 30, and the property of transfer of the tape-like chip to the skin was assessed in 5 stages.
The result is shown in Table 16.
[Table 16]

| Shape | Dimension | Area of Adhesives | Ease of detachment |
|---|---|---|---|
| 1) square shape 1 piece | 10mm x 10mm x 1 piece | 100 mm$^2$ | + |
| 2) triangle shape 4 pieces | 5 mm x 5 mm x 4 piece | 100 mm$^2$ | ++ |
| 3) linear shape 2 pieces (parallel line) | 20mm x 2.5mm x 2 piece | 2 100 mm$^2$ | +++ |
| 4) linear shape 2 pieces (V-shape) | 20mm x 2.5mm x 2 piece | 100 mm$^2$ | ++++ |
| [Note]<br>- : Detachment is impossible<br>+ : Detachment is possible but difficult<br>++ : Detachment is possible<br>+++ : Detachment is smooth<br>++++ : Detachment is very smooth | | | |

As shown in Table 16, it was found out that, even if the area of the adhesive is the same and the adhesion is also the same, the ease of detachment differs depending on the shapes of the adhesive. It was found out that detachment is more likely to occur in the shape where the area of the adhesive is widely dispersed, rather than the shape where the area of the adhesive is focused on one place.

(Test Example 8) Verification of Ease of Transfer of Tape-like Chip to Skin depending on Position where Adhesive is Placed

(1) Comparative Assessment between the case where Adhesive (Double-sided Tape) is Protruded from Periphery of Tape-like Chip and the case where it is Placed on Inner Side from Periphery of Tape-like Chip

As shown in Fig. 40, by using an adhesive (NICETACK NW) having the same shape, there were prepared clog-shaped applicators (devices) in the case where the adhesive was protruded from the periphery of a tape-like chip (Transpore SP1527SP-1) (left drawing) and in the case where the adhesive was placed on the inner side from the periphery of the tape-like chip (right drawing). When a rectangular tape-like chip shown in Fig. 40a) was used, there were prepared a tape-like chip where the adhesive was 0.5 mm protruded from the periphery as shown in the left drawing, and a tape-like chip where the adhesive was placed on the 2 mm inner side from the periphery as shown in the right drawing. In addition, as a dummy for a microneedle, a PP plate (0.8 thick, 10 mm$\phi$) was placed.
The clog-shaped applicators on which the tape-like chips were placed were used. The applicators were pressed against the human forearm by hand, and the property of transfer of the tape-like chip to the skin was assessed. As a result, the one where the adhesive was placed on the inner side from the periphery, as shown in the right drawing, had better property of transfer.
Similarly, by using a circular tape-like chip shown in Fig. 40b), there were prepared the one where the adhesive was 1.0 mm protruded from the periphery as shown in the left drawing, and the one where the adhesive was placed on the 1 mm inner side from the periphery as shown in the right drawing. In addition, as a dummy for a microneedle, a PP plate (0.8 mm thick, 10 mm$\phi$) was placed.
In the same manner as in the above, the property of transfer to the skin was assessed. Then, the one where the adhesive was placed on the inner side from the periphery, as shown in the right drawing, had better property of transfer.

(2) Assessment of Distance of Placed Adhesive from Periphery and Ease of Transfer to Skin

An adhesive (NICETACK NW) was placed on a rectangular tape-like chip (Transpore SP1527SP-1), as shown in Fig. 42. Then, the prepared chips were placed on clog-shaped applicators. It was confirmed how the property of transfer was changed depending on the difference in the distances a and b from the periphery. In addition, as a dummy for a microneedle, a PP plate (0.8 mm thick, 10 mm$\phi$) was placed.
The applicators were pressed against the human forearm by hand, and the property of transfer of the tape-like chip to the skin was assessed. The result is shown in the following Table 17.
[Table 17]

| Distance (mm) from periphery of tape-like chip to adhesive (double-sided tape) | | Property of transfer to skin (Transferred or not) |
|---|---|---|
| Distance a | Distance b | |
| 0.5 | | Not transferred |
| | 0.2 | Transferred |
| | 0.5 | Transferred |
| | 1.0 | Transferred |
| | 2.0 | Transferred |

As shown in the result of Table 17, when the adhesive (double-sided tape) was placed on the inner side from the periphery of the tape-like chip even if only slightly, detachment from the device was likely to occur, and the property of transfer to the skin was high.
From the results of test examples described above, in order to fix a tape-like chip to an applicator (device) and allow the tape-like chip to be transferred to the skin easily, it is possible to select control of the adhesion of an adhesive (double-sided tape, etc.), or the shape of the adhesive and the placed position of the adhesive. In particular, it was found out that the placed position of the adhesive is preferably the inner side from the periphery (a distance from the periphery is 0.1 mm or more) of the tape-like chip.

INDUSTRIAL APPLICABILITY

The device of the present invention is a microneedle device which is easily portable and allows for easy puncturing, so that it can be easily dealt with by ordinary people. Accordingly, the device can be applied to wide uses such as self-injection of insulin etc., and vaccine injection at the time of a pandemic. In addition, since the skin surface rises perpendicularly, the device hardly causes breakage of small needles of the microneedle, and ensures accurate puncturing. Thus, the device ensures accurate injection of drugs. Further, the tape-like chip easily remains on the skin, while including the microneedle. As a result, a microneedle patch, which is easily portable and ensures convenient drug administration to the skin, can be produced.

Claims

1.   A microneedle device for puncturing skin with a microneedle, **characterized in that**

a) two or more skin fixing members are placed on one face of a flat plate,
b) the distance between the skin fixing members is 10 to 70 mm,
c) the skin fixing members each have a prismatic column shape, circular column shape, semicircular column shape (barrel-roof shape), or hemispherical shape,
d) the microneedle is placed on a central portion between/among the skin fixing members, and
e) tip portions of small needles of the microneedle are positioned within the device and less than tip surfaces of the skin fixing members.

2.   The microneedle device according to claim 1, wherein the distance between the skin fixing members is 10 to 40 mm.

3.   The microneedle device according to claim 1 or 2, wherein the skin fixing members are placed at both ends of the flat plate of the device, thereby forming a clog-shape.

4. The microneedle device according to claim 1 or 2, wherein the skin fixing members are placed at four corners of the flat plate of the device.

5. The microneedle device according to claim 1 or 2, wherein the skin fixing members are placed at three places of the flat plate of the device.

6. The microneedle device according to claim 4 or 5, wherein the skin fixing members are prismatic columns, circular columns, or hemispheres.

7. The microneedle device according to any of claims 1 to 7, wherein the prismatic columns are rectangular columns.

8. The microneedle device according to claim 4 or 5, wherein the skin fixing members are hemispheres.

9. The microneedle device according to any of claims 1 to 8, wherein a protrusion (structure) for pressing is placed on a central portion of the flat plate.

10. The microneedle device according to claim 9, wherein the protrusion has a circular column shape or hemispherical shape.

11. The microneedle device according to any of claims 1 to 8, wherein an arched structure is placed on the flat plate as a protrusion (structure) for pressing.

12. The microneedle device according to any of claims 1 to 11, on which a tape-like chip for adhering to skin is placed.

13. The microneedle device according to claim 12, wherein the tape-like chip has the following characteristics:

   a) the tape-like chip is of 1 to 50 cm$^2$,
   b) a support of the tape-like chip and the device are adhered together with an adhesive, and
   c) a microneedle of 5 to 15 mm$\phi$ is placed on a central portion of the tape-like chip.

14. The microneedle device according to claim 12 or 13, wherein the adhesion of the adhesive is 85 to 90% or less of that of an adhesive for adhering to the skin.

# Fig. 1

（1）

Clog applicator
side view

b

a

Clog applicator
bottom view

Clog applicator
top view

（2）

5 mm
prismatic
column

Four-corner applicator
side view

b

a

Four-corner applicator
bottom view

Four-corner applicator
top view

（3）

Three-point applicator
side view

a

a

Three-point applicator
bottom view

Three-point applicator
top view

# Fig. 2

(1)

Perpendicular placement of applicator

b

a

(2)

Parallel placement of applicator

b

a

# Fig. 3

Clog applicator
Skin elevation height (placement on hand: perpendicular)

# Fig. 4

Clog applicator
Skin elevation height (placement on hand: parallel)

# Fig. 5

Four-corner applicator
Skin elevation height

(y-axis) Skin elevation height (mm): 0, 5, 10, 15, 20
(x-axis) side average (mm): 0, 10, 20, 30, 40, 50

Legend:
◆ square
■ rect-
angle

# Fig. 6

Three-point applicator
Skin elevation height

(y-axis) Skin elevation height (mm): 0, 2, 4, 6, 8, 10, 12
(x-axis) side a (mm): 0, 10, 20, 30, 40

Legend:
◆ equilateral
triangle
— linear
equilateral
triangle

# Fig. 7

(1)
Side view

30mm

5mm

3mm

2mm

40mm

(2)
Bottom view

40mm

30mm

5mm

## Fig. 8

# Fig. 9

(1)
Side view

(2)
Bottom view

## Fig. 10

## Fig. 11

Fig. 12

Fig. 13

Fig. 14

Fig. 15

# Fig. 16

Push down
with 5 mmφ rod

This angle is
measured

15mm

Parallelism measurement jig
is adhered to hand

(2)

8 mmφ hole
to pass jig rod through

Top view

Rod of parallelism
measurement jig
(3.5 mmφ circular column)

15mm

Placed on

15mm

Skin fixing member

# Fig. 17

(1)

手

Push with rod

(2)

When skin does not conform
to the applicator

Angle changes from right angle

Hand

(3)

When skin conforms
to the applicator

Angle remains right angle

Hand

# Fig. 18

# Fig. 19

*Wappen* applicator

(1)　　　Opening　25 mm $\phi$　　　　　(sectional view)
　　　　　　Hight　　7 mm

7mm

25mm

(2)　　　　　　　　　　　　　　　　　　(plan view)

Cutting plane of
the applicator

# Fig. 20

Support

Weaker adhesive

Skin fixing member

Microneedle

Strong adhesive (for skin)

# Fig. 21

Push down with finger

Rat skin

SIS rubbery flat plate

# Fig. 22

Clog
applicator

7mm

18mm 30mm

Hollow

Center line

40mm

3.5mm Φ

Parallelism
measurement
jig

7mm

15mm

This angle
is measured

Push down with
pushing rod

Pushing
position
is shifted

Pushing rod is
shifted from the center

# Fig. 23

(a)

(b)

30mm

30mm

40mm

10mm

3mm

3mm

30mm

# Fig. 24

(a)

(b)

30mm

30mm

40mm

10mm

3mm

3mm

30mm

# Fig. 25

30mm

44mm

6mm
30mm
10mm

# Fig. 26

# Fig. 27

Load cell

Tension test jig

0.1 mm x 250 mm
PET sheet

PP plate A
5 mm thickness

Adhesive A

PP plate B
5 mm thickness

Adhesive B

PP plate C
5 mm thickness

# Fig. 28

Load cell

Tension test jig

0.1 mm x 250 mm PET sheet

PP plate A
5 mm thickness

Adhesive A

PP plate B
5 mm thickness

Adhesive B

Human forearm

# Fig. 29

Adhesive area and tensile stress (N)

- ◆ Tensile stress (N) PP
- ▦ Tensile stress (N) Human forearm
- — Linear (Tensile stress (N) PP)
- — Linear (Tensile stress (N) Human forearm)

$y = 13.966x + 2.3998$
$R^2 = 0.9892$

$y = 1.1637x - 0.0557$
$R^2 = 0.9927$

Tensile stress (N)

Adhesive area (cm^2)

# Fig. 30

Load cell

Tension test jig

CATHEREEP FS roll
23 mm$\phi$

Adhesive A
NICETACK NW-40
5mm x 5mm x 7mm

PP plate A   5 mm thickness

Adhesive A

CATHEREEP FS roll
23 mm$\phi$

Human forearm

# Fig. 31

Load cell

Tension test jig

CATHEREEP FS roll
23 mm$\phi$

Adhesive A
NICETACK NW-40
5mm x 5mm x 7mm

0.1mm x 250mm
PET sheet

PP plate A   5 mm thickness

Adhesive A

CATHEREEP FS roll
23 mm$\phi$

PP plate

# Fig. 32

# Fig. 33

(a)  (b)  (c)  Tag

Skin  Skin  Skin

# Fig. 34

(a)

(b)

(c)

(d)

(e)

(f)

(g)

(h)

(i)

(j)

(k)

(l)

(m)

# Fig. 35

1)

CATHEREEP
FS roll

NICETACK
NW-15
10m x 10mm

2)

CATHEREEP
FS roll

NICETACK
NW-15
5m x5 mm x 4 pieces

3)

CATHEREEP
FS roll

NICETACK
NW-15
20 m x 2.5 mm
 x 2 pieces

4)

CATHEREEP
FS roll

NICETACK
NW-15
20 m x 2.5 mm
 x 2 pieces

# Fig. 36

Fig. 37

# Fig. 38

Nichiban Co., Ltd.
NICETACK NW-R
10mm x 7.5mm

Nichiban Co., Ltd.
NICETACK NW-R
10mm x 5mm

Nichiban Co., Ltd.
NICETACK NW-R
20mm x maximum width 5mm

Nichiban Co., Ltd.
NICETACK NW-R
base 5mm x height 5mm

Nichiban Co., Ltd.
NICETACK NW-R
base 5mmxheight 5mm

Nichiban Co., Ltd.
NICETACK NW-R
10mm x 5mm

Each adhesive plaster is
30 x 15 mm
Transpore 1527SP-1, by 3M

Nichiban Co., Ltd.   NICETACK NW-R
base 5mm x height 5mm +   5mm x 15mm
  + base 5mm x height 5mm

Nichiban Co., Ltd.   NICETACK NW-R
base 5mm x height 10mm x 2

# Fig. 39

Nichiban Co., Ltd.
NICETACK NW-R
5 mm x 25 mm

Nichiban Co., Ltd.
NICETACK NW-R
5 mm x 20 mm

Nichiban Co., Ltd.
NICETACK NW-R
$\phi$ 5 mm

Nichiban Co., Ltd.
NICETACK NW-R
$\phi$ 5 mm

Each adhesive plaster is
30 x 15 mm
Transpore 1527SP-1, by 3M

# Fig. 40

a)

0.5mm    2mm

Nichiban Co., Ltd.
NICETACK NW
10 mm x 7.5 mm

3M, Transpore
1527SP-1
30 x 15 mm

b)    1mm    1mm

Nichiban Co., Ltd.
NICETACK NW
base 5mm x height 5mm

3M, Transpore
1527SP-1
25 mm$\phi$

# Fig. 41

# Fig. 42

Distance a

Distance b

3M, Transpore
1527SP-1
25 x 15 mm

Nichiban Co., Ltd.
NICETACK NW-R
10 mm x 15 mm

## INTERNATIONAL SEARCH REPORT

| | |
|---|---|
| | International application No. |
| | PCT/JP2010/004903 |

**A. CLASSIFICATION OF SUBJECT MATTER**
*A61M37/00*(2006.01)i

According to International Patent Classification (IPC) or to both national classification and IPC

**B. FIELDS SEARCHED**

Minimum documentation searched (classification system followed by classification symbols)
A61M37/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2008-212588 A (Toppan Printing Co., Ltd.), 18 September 2008 (18.09.2008), claim 1; paragraphs [0022], [0023], [0025], [0038], [0040]; fig. 2 (Family: none) | 1-14 |
| Y | JP 2009-95410 A (Kunihiko MITSUDA), 07 May 2009 (07.05.2009), paragraph [0021]; fig. 2 to 4 & US 2009/0099503 A1 | 1-14 |
| Y | JP 2005-516737 A (Antares Pharma, Inc.), 09 June 2005 (09.06.2005), paragraph [0021]; fig. 3 to 6 & US 2005/0033234 A1 & WO 2003/068290 A2 | 3,5 |

| | | | |
|---|---|---|---|
| ☒ Further documents are listed in the continuation of Box C. | | ☐ See patent family annex. | |

| | | | |
|---|---|---|---|
| * Special categories of cited documents: | | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| | |
|---|---|
| Date of the actual completion of the international search 09 September, 2010 (09.09.10) | Date of mailing of the international search report 21 September, 2010 (21.09.10) |
| Name and mailing address of the ISA/ Japanese Patent Office | Authorized officer |
| Facsimile No. | Telephone No. |

Form PCT/ISA/210 (second sheet) (July 2009)

**EP 2 462 978 A1**

**INTERNATIONAL SEARCH REPORT**

International application No.

PCT/JP2010/004903

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-307380 A (F. Hoffmann-LA Roche AG.), 29 November 2007 (29.11.2007), paragraph [0024]; all drawings & US 2008/0004573 A1 & EP 1857129 A1 | 9-11 |
| Y | JP 2008-543527 A (3M Innovative Properties Co.), 04 December 2008 (04.12.2008), paragraphs [0028], [0029], [0032] & WO 2007/002521 A2 | 12-14 |
| A | JP 2000-37456 A (Terumo Corp.), 08 February 2000 (08.02.2000), fig. 4, 5 (Family: none) | 1-12 |
| A | Microfilm of the specification and drawings annexed to the request of Japanese Utility Model Application No. 107390/1987(Laid-open No. 12538/1989) (Kao Corp.), 23 January 1989 (23.01.1989), fig. 1, 2 (Family: none) | 6-8 |
| P,A | WO 2009/107806 A2 (Medrx Co., Ltd.), 03 September 2009 (03.09.2009), entire text; all drawings (Family: none) | 1-14 |

Form PCT/ISA/210 (continuation of second sheet) (July 2009)

60

## REFERENCES CITED IN THE DESCRIPTION

**Patent documents cited in the description**

- JP 2006149818 A
- JP 2008520369 A
- JP 2008543527 A

- WO 2008069566 A
- JP 2008535587 A
- JP 2009053749 W